# EUROPEAN PATENT APPLICATION

(11) **EP 3 009 122 A1**
(43) Date of publication of application: **20.04.2016**
(21) Application number: 14811676.7
(22) Date of filing: 10.06.2014
(51) Int. Cl.: A61K 8/19, A61K 8/24, A61K 8/41, A61K 8/81, A61K 8/87, A45D 31/00

(54) **ARTIFICIAL NAIL REMOVAL METHOD, ARTIFICIAL NAIL COMPOSITION, ARTIFICIAL NAIL, ARTIFICIAL NAIL FORMING METHOD, AND NAIL ART KIT**

(30) Priority: 12.06.2013 JP 2013123412
(71) Applicant: FUJIFILM Corporation, Tokyo 106-8620 (JP)
(72) Inventor: OOHASHI Hidekazu, Haibara-gun Shizuoka 421-0396 (JP)
(74) Representative: Hoffmann Eitle
(86) International application number: PCT/JP2014/065296
(87) International publication number: WO 2014/199966

(57) **Abstract**

An object of the invention is to provide an artificial nail removal method by which the burden on the fingertip or nail is reduced without compromising the merit of the glossiness of nail decorations, and without using acetone at the time of removal. Another object of the invention is to provide a nail art kit and an artificial nail composition that are used for the artificial nail removal method, and an artificial nail that uses the artificial nail composition.

Disclosed is an artificial nail removal method including a step of applying an artificial nail composition on or above the nail of a human being or an animal, or on or above a support to form a coating film; a step of drying and/or exposing to light the coating film, and thereby forming an artificial nail; and a removal step of removing the artificial nail by bringing the artificial nail into contact with a removal liquid, in which the artificial nail composition includes (Component A) a compound having an ethylenically unsaturated group and an acid group, and/or (Component B) a polymer having an acid group, and the removal liquid is an aqueous solution having a pH of from 8 to 11.

## Description

### BACKGROUND OF THE INVENTION

### 1. Field of the Invention

The present invention relates to an artificial nail removal method, an artificial nail composition, an artificial nail, an artificial nail forming method, and a nail art kit.

### 2. Description of the Related Art

In recent years, the popularity for nail art that provides a design to apply to the nails of hands or feet is ever increasing, and the technology for forming artificial nails (fake nails, nail tips, and the like) made of synthetic resins on nails (natural nails) has been developed (see JP2004-275736A). Particularly, recently, a nail decoration called artificial nail, which is used by applying a gel-like decorative curable composition including a urethane-based resin and a photopolymerizable monomer on the nail, and curing the composition by irradiating the composition with ultraviolet radiation, is attracting more attention due to the reason that the nail decoration provides a clear finish, is long-wearing and highly adhesive to the nail, and is odorless as in the case of acrylic resins.

When this artificial nail is removed, a method of scratching the surface of the artificial nail by scraping with a coarse file (nail file), thus making it easy for a removal liquid to penetrate, subsequently mounting cotton that has been soaked with an organic solvent such as acetone (removal liquid) on the artificial nail, wrapping this artificial nail and cotton with aluminum foil or the like, leaving the artificial nail to stand so as to swell the artificial nail, and then tearing off this swollen artificial nail using a wooden spatula or the like, has been hitherto mainly used.

Furthermore, regarding the conventional artificial nail compositions, the compositions described in JP2004-275736A, JP2000-256134A, and JP2002-505915A are known.

### SUMMARY OF THE INVENTION

An object of the present invention is to provide an artificial nail removal method by which the burden on the fingertips or nails is reduced without compromising the merit of the glossiness of nail decorations, and without using acetone at the time of removal. Another object of the invention is to provide a nail art kit and an artificial nail composition that are used for the artificial nail removal method, and an artificial nail that uses the artificial nail composition.

The objects described above have been achieved by the means described in the following items <1>, <15>, <17>, and <22> to <24>. These are disclosed below together with items <2> to <14>, <16>, and <18> to <21>, which are preferred embodiments.
<1> An artificial nail removal method, including a step of applying an artificial nail composition on or above the nail of a human being or an animal, or on or above a support to form a coating film; a step of forming an artificial nail by drying and/or exposing the coating film; and a removal step of removing the artificial nail by bringing the artificial nail into contact with a removal liquid, in which the artificial nail composition includes (Component A) a compound having an ethylenically unsaturated group and an acid group, and/or (Component B) a polymer having an acid group, and the removal liquid is an aqueous solution having a pH of from 8 to 11 (preferably from 8.5 to 10.9, more preferably from 8.7 to 10.8, and even more preferably from 9 to 10.7);
<2> The artificial nail removal method according to <1>, wherein Component A is represented by the following Formula (1): 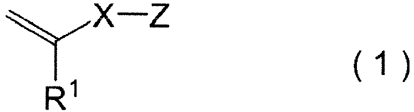 wherein in Formula (1), R¹ represents a hydrogen atom or a monovalent organic group; X represents a single bond or a divalent linking group; and Z represents an acid group;
<3> The artificial nail removal method according to <2>, wherein in the above Formula (1), Z represents a carboxylic acid group;
<4> The artificial nail removal method according to any one of <1> to <3>, wherein Component B is an acrylic polymer and/or a urethane-based polymer;
<5> The artificial nail removal method according to any one of <1> to <4>, wherein Component B is a polymer having a carboxylic acid group;
<6> The artificial nail removal method according to <5>, wherein the polymer having a carboxylic acid group is an acrylic polymer containing a monomer unit represented by the following Formula (2): 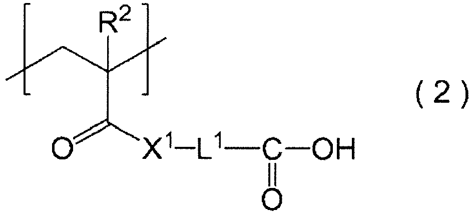 wherein in Formula (2), R² represents a hydrogen atom, a methyl group, or a -CH₂Y¹; Y¹ represents a hydroxyl group, a halogeno group, -OC(=O)R³, or -NR³C(=O)R⁴; X¹ represents -O- or -NR³-; R³ and R⁴ each represent a hydrogen atom, an alkyl group having 1 to 6 carbon atoms, or an aryl group having 6 to 12 carbon atoms; and L¹ represents a divalent linking group;
<7> The artificial nail removal method according to <5>, wherein the polymer having a carboxylic acid group is a urethane-based polymer having a monomer unit represented by the following Formula (3): 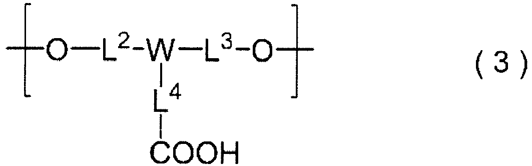 wherein in Formula (3), L², L³ and L⁴ each independently represent a divalent linking group; W represents N or CR³; and R³ represents a hydrogen atom or a monovalent group;
<8> The artificial nail removal method according to any one of <1> to <7>, wherein the removal liquid includes a pH buffering agent;
<9> The artificial nail removal method according to <8>, wherein the pH buffering agent includes at least one selected from the group consisting of a carbonate, a hydrogen carbonate, a phosphate, a borate, and an organic amine compound;
<10> The artificial nail removal method according to <8> to <9>, wherein the pH buffering agent includes a carbonate and a hydrogen carbonate;
<11> The artificial nail removal method according to any one of <1> to <10>, wherein the removal step is a step of removing the artificial nail by immersing the artificial nail in the removal liquid;
<12> The artificial nail removal method according to any one of <1> to <10>, wherein the removal step is a step of removing the artificial nail by spraying the removal liquid to the artificial nail;
<13> The artificial nail removal method according to any one of <1> to <10>, wherein the removal step is a step of removing the artificial nail by wrapping the artificial nail with cotton that has been soaked with the removal liquid;
<14> The artificial nail removal method according to any one of <1> to <13>, wherein the artificial nail composition is used in a base layer;
<15> An artificial nail composition, including (Component A') a compound represented by the following Formula (1'): 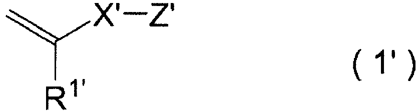 wherein in Formula (1'), R^{1'} represents a hydrogen atom or a monovalent organic group; X' represents a divalent linking group; the smallest number of atoms connecting Z' with the carbon atom to which R^{1'} is bonded is 5 or more; and Z' represents an acid group;
<16> The artificial nail composition according to <15>, wherein in Formula (1'), Z' represents a carboxylic acid group;
<17> An artificial nail composition, including (Component B) a polymer having an acid group;
<18> The artificial nail composition according to <17>, wherein Component B is an acrylic polymer and/or a urethane-based polymer;
<19> The artificial nail composition according to <17> or <18>, wherein Component B is an acrylic polymer containing a monomer unit represented by the following Formula (2): 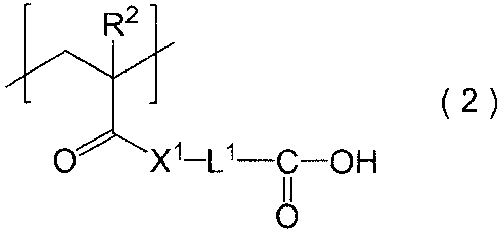 wherein in Formula (2), R² represents a hydrogen atom, a methyl group, or -CH₂Y¹; Y¹ represents a hydroxyl group, a halogeno group, -OC(=O)R³, or -NR³C(=O)R⁴; X¹ represents -O- or -NR³-; R³ and R⁴ each represent a hydrogen atom, an alkyl group having 1 to 6 carbon atoms, or an aryl group having 6 to 12 carbon atoms; and L¹ represents a divalent linking group;
<20> The artificial nail composition according to <17> or <18>, wherein Component B is a urethane-based polymer having a monomer unit represented by the following Formula (3): 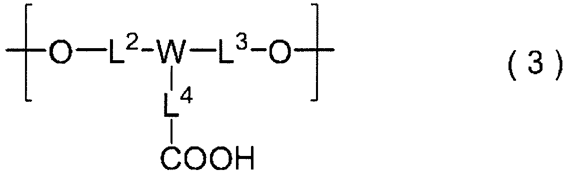 wherein in Formula (3), L², L³, and L⁴ each independently represent a divalent linking group; W represents N or CR³; and R³ represents a hydrogen atom or a monovalent group;
<21> The artificial nail composition according to any one of <15> to <20>, further including (Component D) a photopolymerization initiator;
<22> An artificial nail having a layer formed by drying and/or exposing to light the artificial nail composition according to any one of <15> to <21>;
<23> An artificial nail forming method, including a step of applying the artificial nail composition according to any one of <15> to <21> on or above the nail of a human being or an animal, or on or above a support to form a coating film; and a step of forming an artificial nail by drying and/or exposing to light the coating film; and
<24> A nail art kit including an artificial nail composition and a removal liquid, in which the artificial nail composition includes (Component A) a compound having an ethylenically unsaturated group and an acid group; and/or (Component B) a polymer having an acid group, and the removal liquid is an aqueous solution having a pH of from 8 to 11.

According to the invention, an artificial nail removal method by which the burden on the fingertip or nail is reduced without compromising the merit of the glossiness of nail decorations, and without using acetone at the time of removal, can be provided. Furthermore, a nail art kit and an artificial nail composition that are used for the artificial nail removal method, and an artificial nail that uses the artificial nail composition can be provided.

### BRIEF DESCRIPTION OF THE DRAWING

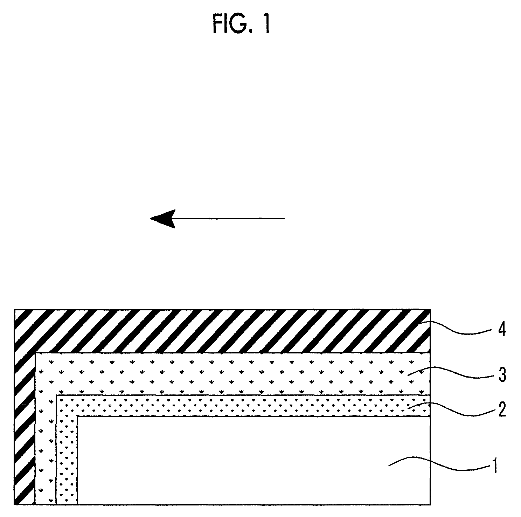

Fig. 1 is a cross-sectional diagram illustrating the configuration of an artificial nail suitable for the invention.

### DESCRIPTION OF THE PREFERRED EMBODIMENTS

Hereinafter, the invention will be described in detail.

In the present specification, the description "xx to yy" represents a value range including xx and yy. Also, "(Component A) a compound having an ethylenically unsaturated group and an acid group" or the like may be simply referred to as "Component A" or the like.

According to the invention, "% by mass" and "% by weight" have the same meaning, and "parts by mass" and "parts by weight" have the same meaning.

In the present specification, regarding the indication of a group in a compound represented by a formula, in a case where it is not described whether the group is substituted or unsubstituted, if the relevant group is capable of further having a substituent, the relevant group is meant to include an unsubstituted group as well as a substituted group unless particularly stated otherwise. For example, in an explanation of a formula, when it is described, "R represents an alkyl group, an aryl group, or a heterocyclic group", this means that "R represents an unsubstituted alkyl group, a substituted alkyl group, an unsubstituted aryl group, a substituted aryl group, an unsubstituted heterocyclic group, or a substituted heterocyclic group." Furthermore, in the present specification, the term (meth)acryl indicates a concept including both acryl and methacryl, and the same also applies to (meth)acrylate and the like.

Furthermore, a polymer according to the present specification is meant to include a copolymer and an oligomer as well.

According to the invention, a combination of preferred embodiments is a more preferred embodiment.

The artificial nail removal method of the invention includes a step of applying an artificial nail composition on or above the nail of a human being or an animal, or on or above a support to form a coating film; a step of forming an artificial nail by drying and/or exposing to light the coating film; and a removal step of removing the artificial nail by bringing the artificial nail into contact with a removal liquid, in which the artificial nail composition includes (Component A) a compound having an ethylenically unsaturated group and an acid group, and/or (Component B) a polymer having an acid group, and the removal liquid is an aqueous solution having a pH of from 8 to 11.

The inventors of the present invention conducted a thorough investigation, and as a result, the inventors found that an artificial nail formed using an artificial nail composition containing Component A and/or Component B can be removed by an aqueous solution having a pH of from 8 to 11, thus completing the invention.

The mechanism by which these effects are manifested is not clearly known; however, the mechanism is partially speculated as follows.

An artificial nail formed using an artificial nail composition containing Component A and/or Component B contains acid groups. It is speculated that by having acid groups, the artificial nail can be removed by an aqueous solution having a pH of from 8 to 11, which is an alkaline aqueous solution. Meanwhile, the artificial nail can also be removed according to a conventional removal method of using an organic solvent; however, when the artificial nail is removed using a less irritating aqueous solution having a pH of from 8 to 11, the damage to the nail or skin is reduced, and the problem of volatile organic compounds (VOC) is solved.

The artificial nail according to the invention refers to a layer formed on or above the nail of a human being or an animal for the purpose of cosmetic decoration and/or protection. Also, for example, the support may be a resin substrate (nail tip) having an arbitrary shape intended for cosmetic decoration and/or protection of the nail.

Meanwhile, the "nail of a human being or an animal, and a support" is also simply referred to as "nail".

The shape of the artificial nail is not particularly limited, and the artificial nail may be formed into any desired shape. For example, the artificial nail may be formed so as to cover the surface of the nail, may be formed on a portion of the nail, or may be formed into a shape larger than the nail using a nail form or the like for the extension of the nail.

Meanwhile, the artificial nail composition of the invention is applied on or above the nail of a human being or an animal, or on or above a support; however, the artificial nail composition may be applied directly on the nail, or may be applied on another layer that has been already formed, without any particular limitations. Among these, as will be described below, it is more preferable that the artificial nail composition is applied as a layer that is in contact with the nail (base layer).

Furthermore, regarding the artificial nail composition of the invention, the thickness can be controlled by application. The thickness is not particularly limited as long as the thickness is in the range that can be generally adopted by an artificial nail; however, from the viewpoint of practical usability and removability, the thickness is preferably in the range of 20 to 1,500 µm.

### (Artificial nail composition)

First of all, the artificial nail composition that is used for the artificial nail removal method of the invention (may also be referred to the artificial nail composition of the invention) is explained.

The artificial nail composition of the invention can be suitably used for any of a primer layer, a base layer, a color layer, and/or a top layer in an artificial nail.

Among them, from the viewpoint of removability, it is preferable that the layer formed using the artificial nail composition of the invention is in contact with the nail, that is, the layer is a base layer.

Also, a primer layer, a base layer, a color layer, and/or a top layer may be formed separately as an upper layer of the artificial nail layer formed using the artificial nail composition of the invention (surface on the opposite side of the nail) or as a lower layer of the artificial nail layer formed using the artificial nail composition of the invention (surface between the artificial nail layer and the nail), for the purpose of providing color, gloss and adhesiveness.

The artificial nail composition of the invention can be suitably used as a photocurable artificial nail composition ("artificial nail composition for gel nail") or as an artificial nail composition for a manicure liquid.

Furthermore, since the artificial nail composition of the invention has excellent removability even if curing is achieved by exposure to light, the artificial nail composition of the invention can be particularly suitably used as a photocurable artificial nail composition.

The photocurable artificial nail composition is an artificial nail composition that can be cured by active light rays. "Active light rays" is a radiation capable of providing energy that generates an initiator species in the artificial nail composition when the composition is irradiated therewith, and examples include ultraviolet radiation and visible light. Among them, from the viewpoints of curing sensitivity and easy availability of the apparatus, ultraviolet radiation is particularly preferred. Therefore, regarding the photocurable artificial nail composition, an artificial nail composition that can be cured by irradiating the composition with ultraviolet radiation as an active light ray, is preferred.

In the following, the various components that can be used in the artificial nail composition of the invention are explained.

### (Component A) Compound having ethylenically unsaturated group and acid group

It is preferable that the artificial nail composition of the invention includes (Component A) a compound having an ethylenically unsaturated group and an acid group.

The molecular weight (in the case of having a molecular weight distribution, weight average molecular weight) of Component A is preferably less than 3,000, preferably from 72 to 2,000, preferably from 86 to 1,500, and more preferably from 144 to 1,000.

When the molecular weight of Component A is in the range described above, excellent removability, curability and adhesiveness are obtained.

Regarding the Component A used in the invention, any compound having both an ethylenically unsaturated bond and an acid group can be suitably used. Examples of the acid group that may be contained in Component A include a carboxylic acid group (a carboxy group, -COOH), a sulfonic acid group (a sulfo group, -SO₃H) a sulfuric acid group (-OSO₂(OH)), a phosphoric acid group (-OPO(OH)₂), a phosphonic acid group (a phosphono group, -PO(OH)₂), a phenolic hydroxyl group, a sulfonamide group (-SO₂-NR²₂), and an imide group (-C(=O)-NR²-C(=O)-). Here, each R² independently represents a hydrogen atom or a monovalent organic group, and the monovalent organic group is preferably an alkyl group having 1 to 6 carbon atoms or an aryl group having 6 to 10 carbon atoms. Among these, the acid group that can be contained by Component A is preferably a carboxylic acid group (-COOH), a sulfonic acid group (-SO₃H), a phosphoric acid group (-OPO(OH)₂), or a phosphonic acid group (-PO(OH)₂); more preferably a carboxylic acid group or a phosphoric acid group; and particularly preferably a carboxylic acid group.

Meanwhile, the acid group may form a salt, and examples thereof include alkali metal salts (for example, a lithium salt, a sodium salt, and a potassium salt), alkaline earth metal salts (for example, a magnesium salt and a calcium salt), and organic cation salts (for example, an ammonium salt).

Component A is a compound having one or more ethylenically unsaturated groups, and may be a monofunctional ethylenically unsaturated compound, or may be a polyfunctional ethylenically unsaturated compound. Component A is preferably a compound having 1 to 6 ethylenically unsaturated bonds, more preferably a compound having 1 to 4 ethylenically unsaturated bonds, even more preferably a compound having 1 to 3 ethylenically unsaturated bonds, particularly preferably a compound having 1 or 2 ethylenically unsaturated bonds, and most preferably a compound having one ethylenically unsaturated bond. That is, it is most preferable that Component A is a monofunctional ethylenically unsaturated compound.

Component A is preferably a compound represented by the following Formula (1) and more preferably a compound represented by Formula (1'): 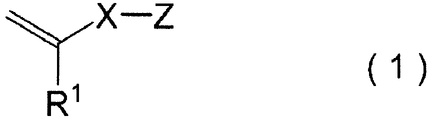 wherein in Formula (1), R¹ represents a hydrogen atom or a monovalent organic group; X represents a single bond or a divalent organic group; and Z represents an acid group; and 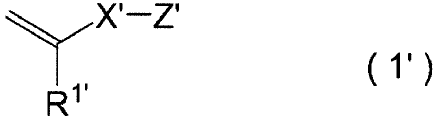 wherein in Formula (1'), R^{1'} represents a hydrogen atom or a monovalent organic group; X' represents a divalent linking group; the minimum number of atoms connecting Z' with the carbon atom to which R^{1'} is bonded is 5 or more; and Z' represents an acid group.

Specific examples of the monovalent organic group represented by R¹ and R^{1'} include an alkyl group having 1 to 18 carbon atoms, an aryl group having 6 to 18 carbon atoms, an alkenyl group having 2 to 18 carbon atoms, -C(=O)OR², -C(=O)NR³R⁴, -CH₂OC(=O)R², -CH₂NHC(=O)R², and -CH₂OR² (wherein R² represents a hydrogen atom, an alkyl group having 1 to 18 carbon atoms, an aryl group having 6 to 18 carbon atoms, or an alkenyl group having 2 to 18 carbon atoms; and R³ and R⁴ each independently represent hydrogen, an alkyl group having 1 to 18 carbon atoms, an aryl group having 6 to 18 carbon atoms, or an alkenyl group having 2 to 18 carbon atoms). Preferred examples of R¹ and R^{1'} include a hydrogen atom, an alkyl group having 1 to 6 carbon atoms, an aryl group having 6 to 12 carbon atoms, -C(=O)OR², -C(=O)NR³R⁴, -CH₂OC(=O)R², -CH₂NHC(=O)R², and -CH₂OR² (wherein R² represents a hydrogen atom, an alkyl group having 1 to 6 carbon atoms, an aryl group having 6 to 12 carbon atoms, or an alkenyl group having 2 to 6 carbon atoms; and R³ and R⁴ each independently represent a hydrogen atom, an alkyl group having 1 to 6 carbon atoms, an aryl group having 6 to 12 carbon atoms, or an alkenyl group having 2 to 6 carbon atoms); more preferred examples include a hydrogen atom, an alkyl group having 1 to 3 carbon atoms, -C(=O)OR², -C(=O)NR³R⁴, -CH₂OC(=O)R², -CH₂NHC(=O)R², and -CH₂OR² (wherein R² represents hydrogen or an alkyl group having 1 to 3 carbon atoms; and R³ and R⁴ each independently represent a hydrogen atom or an alkyl group having 1 to 3 carbon atoms); even more preferred examples include a hydrogen atom, a methyl group, -COOMe, -COOH, -CH₂OC(=O)Me -CH₂OH and -CH₂OMe; and particularly preferred examples include a hydrogen atom or a methyl group. The foregoing abbreviation Me means a methyl group.

X represents a single bond or a divalent linking group; and it is more preferable that X is a divalent linking group. A specific example of the divalent organic group represented by X is a divalent organic group which contains carbon (C) as an essential component, and is formed by appropriately selecting elements from oxygen (O), nitrogen (N), sulfur (S), phosphorus (P), hydrogen (H), halogen (F, Cl, Br, and I), and silicon (Si). Among them, X represents a divalent linking group represented by -A¹-A²-A³-A⁴-A⁵- ... -Aⁿ- (wherein Aⁿ represents an element selected from carbon, oxygen, nitrogen, sulfur, phosphorus, and silicon, all of which may be substituted with a hydrogen atom, a halogen atom, or a substituent; and the substituents carried by the respective elements may be bonded to form a ring), while n is preferably 3 or more, more preferably 4 or more, and particularly preferably 5 or more. Regarding the substituent, a substituent having 1 to 12 carbon atoms is preferred.

That is, in regard to the divalent linking group represented by X, the minimum number of atoms that connect Z (acid group) with the carbon to which R¹ is bonded is preferably 3 or more, more preferably 4 or more, and particularly preferably 5 or more (that is, X is X'). The upper limit is not particularly limited; however, the minimum number of atoms is preferably 30 or less, more preferably 20 or less, and even more preferably 10 or less. Furthermore, Aⁿ is selected from the group consisting of carbon having a hydrogen atom, a halogen atom or a substituent; oxygen (-O); nitrogen which may have a hydrogen atom, a halogen atom or a substituent (-NR- or -N=, wherein R represents a hydrogen atom, a halogen atom, or a substituent); sulfur (-S-); phosphorus having a hydrogen atom, a halogen atom or a substituent; and silicon having a hydrogen atom, a halogen atom or a substituent.

Specific examples of such a divalent organic group include *-C(=O)O-Q-**, *C(=O)NR⁵-Q-**, *-C(=O)O-Q-OC(=O)-Q-**, *-C(=O)NR⁵-Q-OC(=O)-Q-**, *-C(=O)NR⁵-Q-NR⁶-C(=O)-Q-**, *-phenylene-C(=O)O-Q-**, *-phenylene-C(=O)NR⁵-Q-**, *-phenylene-CH₂-O-Q-**, and a phenylene group; however, the invention is not intended to be limited to these. Meanwhile, the symbol * represents the position of bonding between X and the carbon atom to which R¹ is bonded, and the symbol ** represents the position of bonding between X and Z. R⁵ and R⁶ each represent a hydrogen atom, an alkyl group having 1 to 18 carbon atoms, or an aryl group having 6 to 12 carbon atoms. Each Q' independently represents an alkylene group having 1 to 30 carbon atoms which may have a substituent, or an arylene group having 6 to 18 carbon atoms which may have a substituent, and the alkylene group may be any of linear, branched, or cyclic, and may have an ether bond (-0-) between carbon-carbon bonds. Meanwhile, the aforementioned carbon number means the total carbon number including the carbon numbers of substituents.

According to the invention, X is preferably *-C(=O)O-Q-**, -C(=O)NR⁵-Q-**, *-C(=O)O-Q-OC(=O)-Q-**, *-C(=O)NR⁵-Q-OC(=O)-Q-**, *-C(=O)NR⁵-Q-NR⁶-C(=O)-Q-**, *-phenylene-C(=O)O-Q-**, *-phenylene-C(=O)NR⁵-Q-**, or *-phenylene-CH₂-O-Q-**; more preferably *-C(=0)0-Q'-**, *-C(=O)NR⁵-Q'-**, *-C(=O)O-Q'-OC(=O)-Q'-**, or *-C(=O)NR⁵-Q'-OC(=O)-Q'-**; and particularly preferably *-C(=0)O-Q"-**, *-C(=O)NR⁵-Q"-**, *-C(=O)O-Q"-OC(=O)-Q"-**, or -C(=O)NR⁵-Q"-OC(=O)-Q"-. Here, each Q' independently represents an alkylene group having 2 to 25 carbon atoms which may have a substituent, or an arylene group having 6 to 12 carbon atoms which may have a substituent, and the alkylene group may be any of linear, branched, or cyclic, and may have an ether bond between carbon-carbon bonds. Each Q" independently represents an alkylene group having 2 to 9 carbon atoms which may have a substituent or an arylene group having 6 to 12 carbon atoms which may have a substituent, and the alkylene group may be any of linear, branched, or cyclic, and may have an ether bond between carbon-carbon bonds.

Examples of the acid group represented by Z and Z' include -COOH, -SO₃H, -OPO₃H₂, and PO₃H₂, and among them, -COOH or -OPO₃H₂ is preferred, while -COOH is particularly preferred.

According to the invention, Component A is preferably such that an ethylenically unsaturated bond and an acid group are bonded through a certain linking group, and the acid group is preferably a carboxylic acid group.

That is, in Formula (1), it is particularly preferable that R¹ represents a hydrogen atom or a methyl group; the acid group represented by Z is -COOH, and X represents *-C(=O)O-Q"-** or *-C(=O)O-Q"-OC(=O)-Q"-**. Meanwhile, it is particularly preferable that the minimum number of atoms connecting Z with the carbon atom to which R¹ is bonded is 5 or more.

Specific examples of the monomer having an acid group include a (meth)acrylic acid-based monomer having a carboxylic acid group, a styrene-based monomer having a carboxylic acid group, a (meth)acrylic monomer having a sulfonic acid group, a styrene-based monomer having a sulfonic acid group, a (meth)acrylic acid-based monomer having a phosphoric acid group, and other monomers having acid groups.

Examples of the (meth)acrylic acid-based monomer having a carboxylic acid group include acrylic acid, methacrylic acid, itaconic acid, 2-(meth)acryloyloxyethylsuccinic acid, 2-(meth)acryloyloxyethylhexahydrophthalic acid, 2-(meth)acryloyloxyethylphthalic acid, 2-(meth)acryloyloxypropylphthalic acid, 2-(meth)acryloyloxypropylsuccinic acid, 2-(meth)acryloyloxyacetic acid, β-carboxyethy (meth)acrylate, 4-(meth)acryloyloxybutanoic acid, 2-(meth)acryloyloxyethoxyacetic acid, 2-(meth)acryloyloxyethylmaleic acid, 6-(meth)acryloyloxyhexanoate, polyethylene glycol mono(meth)acrylate succinic acid, polyethylene glycol mono(meth)acrylate hexahydrophthalic acid, polyethylene glycol mono(meth)acrylate phthalic acid, polypropylene glycol mono(meth)acrylate succinic acid, polypropylene glycol mono(meth)acrylate hexahydrophthalic acid, and polypropylene glycol mono(meth)acrylate phthalic acid.

Examples of the styrene-based monomer having a carboxylic acid group include 4-carboxystyrene, and examples of the (meth)acrylic monomer having a sulfonic acid group include 3-(meth)acryloyloxypropylsulfonic acid and 2-(meth)acrylamido-2-methyl-1-propanesulfon acid. Examples of the styrene-based monomer having a sulfonic acid group include 4-vinylbenzenesulfonic acid.

Examples of the (meth)acrylic acid-based monomer having a phosphoric acid group include 2-(meth)acryloyloxyethyl phosphate and polyoxyethylene (meth)acrylate phosphate, and examples of the other monomers having acid groups include vinylphosphonic acid and vinylsulfonic acid.

Among the specific examples of the monomer having an acid group, 2-(meth)acryloyloxyethylsuccinic acid, 2-(meth)acryloyloxyethylhexahydrophthalic acid, 2-(meth)acryloyloxyethylphthalic acid, 2-(meth)acryloyloxypropylphthalic acid, 2-(meth)acryloyloxypropylsuccinic acid, β-carboxyethyl (meth)acrylate, 2-(meth)acryloyloxyethylmaleic acid, 6-(meth)acryloyloxyhexanoate, (2-(meth)acryloyloxy)ethoxyacetic acid, 4-(meth)acryloyloxybutanoic acid, polyethylene glycol mono(meth)acrylate succinic acid, polyethylene glycol mono(meth)acrylate hexahydrophthalic acid, polyethylene glycol mono(meth)acrylate phthalic acid, polypropylene glycol mono(meth)acrylate succinic acid, polypropylene glycol mono(meth)acrylate hexahydrophthalic acid, and polypropylene glycol mono(meth)acrylate phthalic acid are particularly preferred from the viewpoint of having excellent removability.

Examples of compounds preferred as Component A are listed below; however, the invention is not intended to be limited by the description of these compounds. 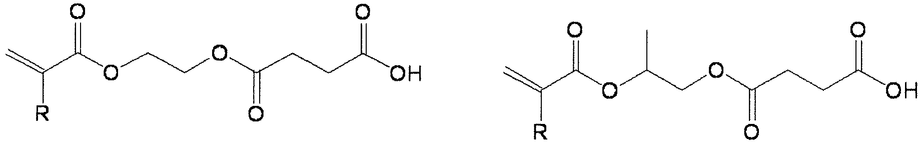 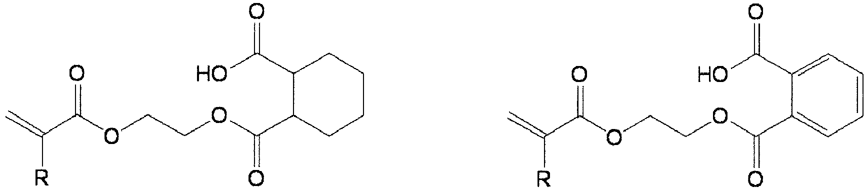 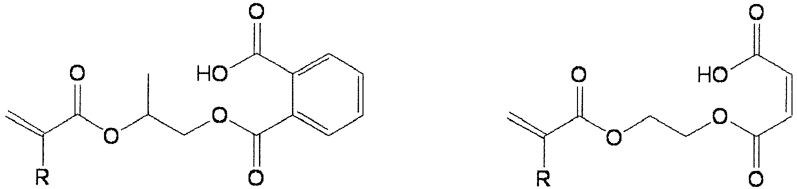 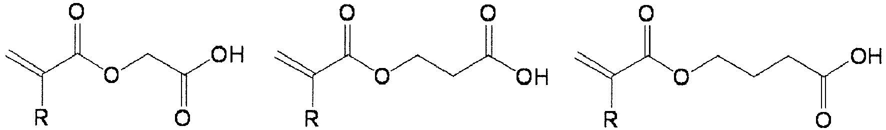 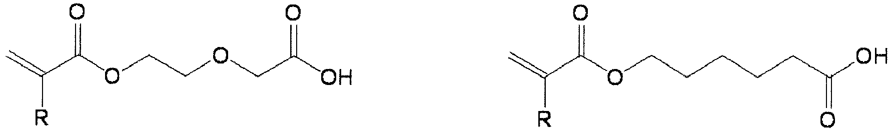 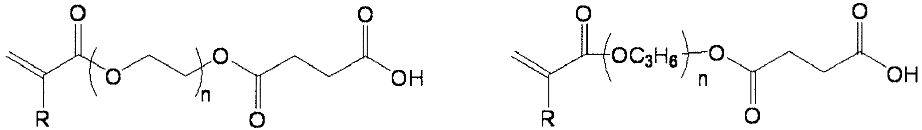 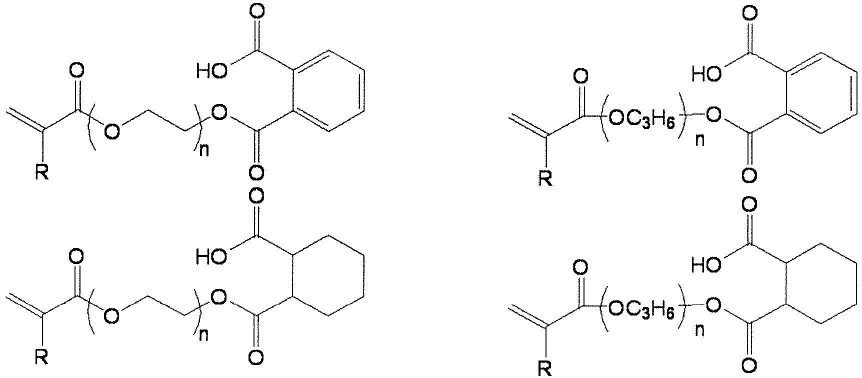 wherein in the formulae, R represents a hydrogen atom or a methyl group; and n represents an integer of from 1 to 10.

Furthermore, regarding the monomer having an acid group, a polyfunctional monomer having an acid group can also be suitably used. Examples of the polyfunctional monomer having an acid group include a (meth)acrylic acid ester of a carboxylic acid having two or more hydroxyl groups (for example, tartaric acid, shikimic acid, or mucic acid), a reaction product of a carboxylic acid having two or more hydroxyl groups and 2-isocyanatoethyl (meth)acrylate, (meth)acrylic acid amide of a carboxylic acid having two or more amino groups (for example, 2,3-diaminopropanoic acid, lysine, ornithine, or cystine), a reaction product of a carboxylic acid having two or more amino groups and 2-isocyanatoethyl (meth)acrylate, a dehydration condensate of a carboxylic acid having two or more in total of a hydroxyl group and an amino group (for example, serine or threonine) and (meth)acrylic acid, and a reaction product of a carboxylic acid having two or more in total of a hydroxyl group and an amino group and 2-isocyanatoethyl (meth)acrylate.

It is more preferable that Component A is liquid at room temperature. Furthermore, it is also preferable that the solubility at room temperature in ethyl acetate is 5% by mass or more. These monomers having an acid group may be used singly, or in combination of two or more kinds thereof. These monomers having an acid group may be used after having some or all of the acid groups neutralized with a base (for example, sodium hydroxide, potassium carbonate, triethylamine, or imidazole).

### (Component B) Polymer having acid group

It is preferable that the artificial nail composition of the invention includes (Component B) a polymer having an acid group.

The structure of Component B is not particularly limited, and Component B may have any arbitrary structure, examples of which include a chain-like structure, a branched structure, a star-shaped structure, a crosslinked structure, and a network structure.

Regarding the polymer having an acid group that is used in the invention, any known polymers having an acid group (for example, an acrylic polymer, a urethane-based polymer, a cellulose-based polymer, an ester-based polymer, an amide-based polymer, a vinyl-based polymer, an ether-based polymer, a styrene-based polymer, a carbonate-based polymer, and a urea-based polymer) can all be suitably used. Among these polymers, an acrylic polymer and a urethane-based polymer are particularly preferred from the viewpoints of removability, strength, cost, and synthesis adaptability.

Examples of the acid group include known acid groups such as a carboxylic acid group, a sulfonic acid group, a phosphoric acid group, a phosphonic acid group, and a sulfuric acid group; however, a carboxylic acid group is particularly preferred from the viewpoints of removability and stability.

Furthermore, the acid group may be positioned at any of a side chain or a chain end of the polymer main chain; however, in a case where the acid group is positioned in a side chain, it is preferable that the side chain contains two or more atoms, more preferably 3 or more atoms, and particularly preferably 4 or more atoms, between the polymer main chain and the acid group. In a case where the acid group is positioned at an end of the main chain, it is preferable that the acid group is positioned at, at least one end of the main chain, and it is more preferable that the acid group is positioned at both ends of the main chain. Furthermore, it is also preferable that the acid group is positioned both at the ends and in a side chain.

It is preferable that the acrylic polymer having an acid group contains a monomer unit represented by the following Formula (2): 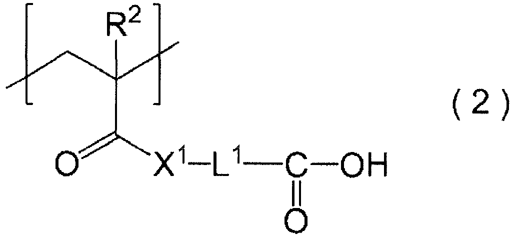 wherein in the formula, R² represents a hydrogen atom, a methyl group, or -CH₂Y¹; Y¹ represents a hydroxyl group, a halogeno group, -OC(=O)R³, or -NR³C(=O)R⁴; X¹ represents -O- or -NR³-; R³ and R⁴ each independently represent a hydrogen atom, an alkyl group having 1 to 6 carbon atoms, or an aryl group having 6 to 12 carbon atoms; and L¹ represents a divalent linking group.

In regard to the divalent linking group represented by L¹, any divalent groups containing carbon as an essential element and composed of oxygen (O), nitrogen (N), sulfur (S), phosphorus (P), hydrogen (H), halogen (F, Cl, Br, and I), or silicon (Si) can all be suitably used. However, the relevant divalent linking group is preferably a divalent linking group that connects X¹ with COOH to be separated by two or more atoms; more preferably a divalent group that connects the two to be separated by three or more atoms [X¹-A¹-A²- ... -Aⁿ-COOH (wherein in the formula, Aⁿ represents an atom; and n represents an integer of 3 or greater)]; and particularly preferably a divalent linking group that connects the two to be separated by four or more atoms [X¹-A¹-A²- ... -Aⁿ-COOH (wherein in the formula, Aⁿ represents an atom; and n represents an integer of 4 or greater)]. It is preferable that the minimum (smallest) number of atoms connecting X¹ with a carboxylic acid group is large, and when such a divalent linking group is used, satisfactory removability is obtained. The upper limit of the minimum number of atoms connecting X¹ with a carboxylic acid group is not particularly limited; however, from the viewpoints of synthesis and removability, the minimum number of atoms is preferably 30 or less, more preferably 20 or less, and even more preferably 10 or less.

Specific examples of L¹ include *-Q-**, *-Q-OC(=O)-Q-**, and *-Q-NR³C(=O)-Q-**; however, the invention is not intended to be limited to these. Meanwhile, the symbol * represents the position of bonding between X¹ and L¹. Each Q' independently represents an alkylene group having 1 to 30 carbon atoms which may have a substituent, or an arylene group having 6 to 18 carbon atoms which may have a substituent, and the alkylene group may be any of linear, branched, or cyclic, and may have an ether bond between carbon-carbon bonds. Meanwhile, the aforementioned carbon number means the total carbon number including the carbon numbers of substituents. R³ represents a hydrogen atom, an alkyl group having 1 to 6 carbon atoms, or an aryl group having 6 to 12 carbon atoms.

According to the invention, L¹ is preferably *-Q-**, *-Q-OC(=O)-Q-**, or *-Q-NR³C(=O)-Q-**; more preferably *-Q'-**, *-Q'-OC(=O)-Q'-**, or *-Q'-NR³C(=O)-Q'-**; and even more preferably *-Q"-**, *-Q"-OC(=O)-Q"-**, or *-Q"-NR³C(=O)-Q"-**. Here, each Q' independently represents an alkylene group having 2 to 24 carbon atoms which may have a substituent or an arylene group having 6 to 12 carbon atoms which may have a substituent, and the alkylene group may be any of linear, branched and cyclic groups, and may have an ether bond between carbon-carbon bonds. Each Q" independently represents an alkylene group having 2 to 18 carbon atoms which may have a substituent, or an arylene group having 6 to 12 carbon atoms which may have a substituent, and the alkylene group may be any of linear, branched, or cyclic, and may have an ether bond between carbon-carbon bonds. Q' is preferably an alkylene group having 2 to 24 carbon atoms, and the alkylene group is preferably a linear or branched group, and may have an ether bond between carbon-carbon bonds. Q" is preferably an alkylene group having 2 to 18 carbon atoms which may have a substituent, and the alkylene group is preferably a linear or branched group, and may have an ether bond between carbon-carbon bonds.

The acrylic polymer having a carboxylic acid group may be an acrylic polymer synthesized using a (meth)acrylic acid-based monomer having a carboxylic acid group. Examples of the (meth)acrylic acid-based monomer having a carboxylic acid group include acrylic acid, methacrylic acid, 2-methacryloyloxyethylsuccinic acid, 2-acryloyloxyethylsuccinic acid, 2-methacryloyloxyethylhexahydrophthalic acid, 2-acryloyloxyethylhexahydrophthalic acid, 2-acryloyloxyethylphthalic acid, 2-methacryloyloxyethylphthalic acid, 2-acryloyloxypropylphthalic acid, 2-methacryloyloxypropylphthalic acid, 2-acryloyloxypropylsuccinic acid, 2-methacryloyloxypropylsuccinic acid, 2-(meth)acryloyloxyacetic acid, β-carboxyethyl (meth)acrylate, 4-(meth)acryloyloxybutanoic acid, 2-(meth)acryloyloxyethoxyacetic acid, 2-methacryloyloxyethylmaleic acid, 6-acryloyloxyhexanoate, polyethylene glycol mono(meth)acrylate succinic acid, polyethylene glycol mono(meth)acrylate hexahydrophthalic acid, polyethylene glycol mono(meth)acrylate phthalic acid, polypropylene glycol mono(meth)acrylate succinic acid, polypropylene glycol mono(meth)acrylate hexahydrophthalic acid, and polypropylene glycol mono(meth)acrylate phthalic acid.

Among these (meth)acrylic acid-based monomers having a carboxylic acid group that are used for the synthesis of acrylic polymers having carboxylic acid groups, 2-methacryloyloxyethylsuccinic acid, 2-acryloyloxyethylsuccinic acid, 2-methacryloyloxyethylhexahydrophthalic acid, 2-acryloyloxyethylhexahydrophthalic acid, 2-acryloyloxyethylphthalic acid, 2-methacryloyloxyethylphthalic acid, 2-acryloyloxypropylphthalic acid, 2-methacryloyloxypropylphthalic acid, 2-acryloyloxypropylsuccinic acid, 2-methacryloyloxypropylsuccinic acid, β-carboxyethyl (meth)acrylate, 2-(meth)acryloyloxyacetic acid, 2-methacryloyloxyethylmaleic acid, 6-acryloyloxyhexanoate, polyethylene glycol mono(meth)acrylate succinic acid, polyethylene glycol mono(meth)acrylate hexahydrophthalic acid, polyethylene glycol mono(meth)acrylate phthalic acid, polypropylene glycol mono(meth)acrylate succinic acid, polypropylene glycol mono(meth)acrylate hexahydrophthalic acid, and polypropylene glycol mono(meth)acrylate phthalic acid are more preferred; and 2-methacryloyloxyethylsuccinic acid, 2-acryloyloxyethylsuccinic acid, 2-acryloyloxypropylphthalic acid, 2-methacryloyloxypropylphthalic acid, β-carboxyethyl (meth)acrylate (acrylic acid dimer), 6-acryloyloxyhexanoate, polyethylene glycol mono(meth)acrylate succinic acid, polyethylene glycol mono(meth)acrylate hexahydrophthalic acid, polyethylene glycol mono(meth)acrylate phthalic acid, polypropylene glycol mono(meth)acrylate succinic acid, polypropylene glycol mono(meth)acrylate hexahydrophthalic acid, and polypropylene glycol mono(meth)acrylate phthalic acid are particularly preferred.

In regard to the synthesis of an acrylic polymer having a carboxylic acid group that is used for the invention, any known acrylic acid ester monomer (for example, methyl acrylate, ethyl acrylate, t-butyl acrylate, or cyclohexyl acrylate), a methacrylic acid ester monomer (for example, methyl methacrylate, ethyl methacrylate, butyl methacrylate, isobutyl methacrylate, or 2-ethylhexyl methacrylate), an acrylamide monomer (for example, acrylamide, N-isopropylacrylamide, N,N-dimethylacrylamide, or acryloylmorpholine), a methacrylamide monomer (for example, methacrylamide), or some other monomer having an ethylenically unsaturated bond (for example, styrene, vinyl acetate, or N-vinylpyrrolidone) may also be used as a copolymer component.

It is preferable that the acrylic polymer having an acid group contains a monomer unit represented by the above Formula (2) at a content of 5 mol% to 50 mol%, more preferably at a content of 10 mol% to 45 mol%, and even more preferably at a content of 15 mol% to 40 mol%.

The urethane-based polymer having a carboxylic acid group is preferably a urethane-based polymer containing a monomer unit represented by the following Formula (3): 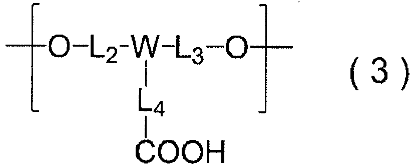 wherein in Formula (3), L², L³, and L⁴ each independently represent a divalent linking group; W represents N or CR³; and R³ represents a monovalent group.

Regarding the divalent linking group represented by L² or L³, any divalent groups containing carbon as an essential element and composed of hydrogen, oxygen, nitrogen, sulfur, phosphorus, halogen, or silicon can all be suitably used. The divalent linking group represented by L² or L³ is preferably an alkylene group having 1 to 12 carbon atoms or an arylene group having 6 to 12 carbon atoms; and more preferably an alkylene group having 1 to 12 carbon atoms. The alkylene group may be any of linear, branched, or cyclic, and may have one or more ether bonds between carbon-carbon bonds. Examples of the divalent linking group include a methylene group, an ethylene group, a propylene group, a phenylene group, -CH₂CH₂-O-CH₂CH₂-, a poly(oxyethylene) group, and a poly(oxypropylene) group; however, the invention is not intended to be limited to these.

Regarding the divalent linking group represented by L⁴, any divalent groups containing carbon as an essential element and composed of hydrogen, oxygen, nitrogen, sulfur, phosphorus, halogen or silicon can all be suitably used. However, the relevant divalent linking group is preferably a divalent group that connects W with COOH to be separated by two or more atoms [W-A¹-A²- ... -Aⁿ-COOH (wherein Aⁿ represents an atom, and n represents an integer of 2 or greater)], more preferably a divalent group that connects the two to be separated by three or more atoms [W-A¹-A²- ... -Aⁿ-COOH (wherein Aⁿ represents an atom, and n represents an integer of 3 or greater)], and particularly preferably a divalent group that connects the two to be separated by four or more atoms [W-A¹-A²- ... Aⁿ-COOH (wherein Aⁿ represents an atom, and n represents an integer of 4 or greater)]. When these divalent linking groups are used, satisfactory removability is obtained.

L⁴ is preferably *-Q-** or *-C(=O)-Q-**. Here, the symbol * represents the position of bonding between W and L⁴. Q represents an alkylene group having 1 to 18 carbon atoms which may have a substituent, or an arylene group having 6 to 12 carbon atoms which may have a substituent, and the alkylene group may be any of linear, branched, or cyclic, and may have an ether bond between carbon-carbon bonds. Meanwhile, the aforementioned carbon number means the total carbon number including the carbon numbers of substituents.

L⁴ is more preferably *-Q'-** or *-C(=O)-Q'-**, and even more preferably *-Q"-** or *-C(=O)-Q"-**. Here, the symbol * represents the position of bonding between W and L⁴. Q' represents an alkylene group having 1 to 12 carbon atoms which may have a substituent, and the alkylene group may be any of linear, branched, or cyclic, and may have an ether bond between carbon-carbon bonds. Meanwhile, the aforementioned carbon number means the total carbon number including the carbon numbers of substituents. Q" represents an alkylene group having 2 to 8 carbon atoms which may have a substituent, and the alkylene group may be any of linear, branched, or cyclic, and may have an ether bond between carbon-carbon bonds. Meanwhile, the aforementioned carbon number means the total carbon number including the carbon numbers of substituents.

W represents N or CR³, and W is preferably N.

R³ represents a monovalent group, and R³ is preferably a hydrogen atom, an alkyl group having 1 to 12 carbon atoms, or an aryl group having 6 to 12 carbon atoms, and more preferably a hydrogen atom or an alkyl group having 1 to 6 carbon atoms.

Examples of a monomer unit represented by Formula (3) are shown below; however, the invention is not intended to be limited to the following specific examples:

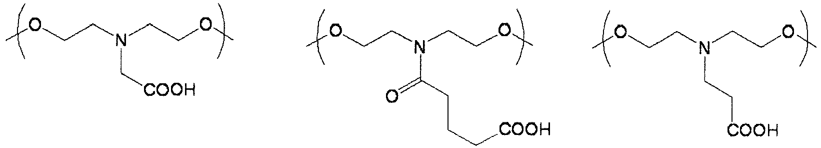

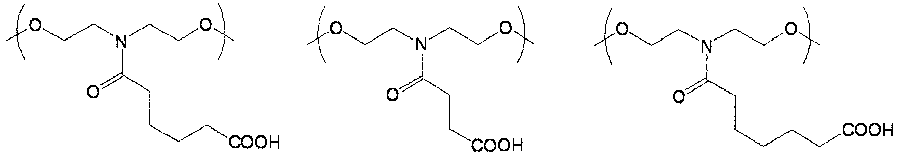

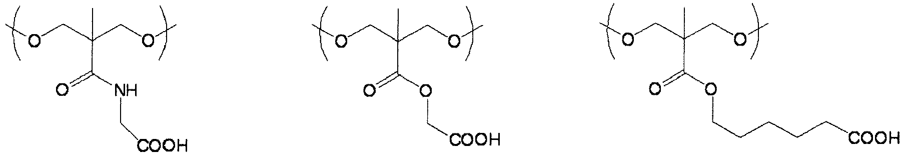

The urethane-based polymer is obtained by a reaction between a carboxyl group-containing diol compound that forms a monomer unit represented by Formula (3) and a polyisocyanate compound.

Examples of the isocyanate compound that may be suitably used include isophorone diisocyanate, hexamethylene diisocyanate, methylenebisphenyl isocyanate, tolylene diisocyanate, 2,2,4-trimethylhexamethylene diisocyanate, 2,4,4-trimethylhexamethylene diisocyanate, and m-xylylene diisocyanate.

Meanwhile, in addition to the carboxyl group-containing diol compound that forms a monomer unit represented by Formula (3), other alcohol compounds may also be used in combination.

Regarding the urethane-based polymer having a carboxylic acid group, a urethane-based polymer having carboxylic acid groups at the chain ends is also preferable.

Examples of the urethane-based polymer having a carboxylic acid group include urethane-based polymers obtainable by reacting a monool compound having a carboxylic acid group (example: glycolic acid, lactic acid, 3-hydroxybutanoic acid, 3-hydroxy-3-methylglutaric acid, malic acid, citric acid, or salicylic acid), a diol compound having a carboxylic acid group (example: 2,2-bis(hydroxymethyl)propionic acid, tartaric acid, 3,5-dihydroxybenzoic acid, or N,N-bishydroxyethylsuccinic acid monoamide), or a polyol compound having a carboxylic acid group (example: shikimic acid, galactaric acid, or gluconic acid), with a known isocyanate compound (isophorone diisocyanate, hexamethylene diisocyanate, methylenebisphenyl isocyanate, tolylene diisocyanate, or the like) and any other arbitrary alcohol compound (methanol, ethanol, 1,4-butanediol, polyethylene glycol, polypropylene glycol, tetraethylene glycol, or the like).

Component B that is used for the invention is intended to include a copolymer and an oligomer as well. The weight average molecular weight of the polymer having an acid group measured by GPC and calculated relative to polystyrene standards is preferably from 3,000 to 1,000,000, more preferably from 4,000 to 500,000, and particularly preferably from 5,000 to 250,000.

When the weight average molecular weight of Component B is in the range described above, it is preferable because long-lastingness and removability of the artificial nail are improved.

The polymer used in the invention may have a radically polymerizable functional group. The radically polymerizable functional group is preferably a (meth)acrylic acid ester group, a (meth)acrylic acid amide group, a styrene group, a vinyl ether group, or a vinyl ester group; more preferably a (meth)acrylic acid ester group or a (meth)acrylic acid amide group; and particularly preferably a (meth)acrylic acid ester group.

According to the invention, when Component B has a carboxyl group as an acid group, the carboxyl group equivalent to Component B is preferably 0.25 to 5.0 meq/g, more preferably 0.5 to 4.0 meq/g, and even more preferably 0.75 to 3.0 meq/g.

The artificial nail composition of the invention includes any of Component A and/or Component B as an essential component. The amount of Component A and Component B used in the artificial nail composition is, as a total amount, preferably from 10% by mass to 95% by mass, more preferably from 20% by mass to 90% by mass, and particularly preferably from 30% by mass to 85% by mass. When Component A and Component B are used in combination, the proportion of Component A to Component B (mass ratio, A/B) is preferably from 1/10 to 10/1, more preferably from 2/10 to 10/2, and particularly preferably from 3/10 to 10/3. When the amount of use described above is employed, removability of the artificial nail film is ameliorated.

Furthermore, the artificial nail composition of the invention may include a polymer other than Component B, for the purpose of enhancing the coating effect or glossiness.

Particularly, when the artificial nail composition of the invention includes Component A, it is preferable that the artificial nail composition includes any kind of polymer component, and it is a preferable embodiment that the artificial nail composition includes a polymer other than Component B.

The polymer other than Component B is preferably an acrylic polymer or a urethane-based polymer, from the viewpoint of the coating effect. Also, for the purpose of enhancing coatability, it is also acceptable that the artificial nail composition includes any well-known polymer which is known as a viscosity adjusting agent (thickening agent or the like).

When the artificial nail composition of the invention does not include Component A but includes Component B, the content of the polymer other than Component B is preferably less than the content of Component B, and the content is more preferably 20% by mass or less, even more preferably 10% by mass or less, and still more preferably 5% by mass or less, relative to the total mass of the artificial nail composition, while it is particularly preferable that the artificial nail composition does not include any polymer other than Component B.

According to the invention, when the artificial nail composition includes Component A, it is also a preferable embodiment that Component B is used as the polymer component.

### (Component C) Other polymerizable compound

It is preferable that the artificial nail composition of the invention includes (Component C) another polymerizable compound. Component C is a polymerizable compound that does not have an acid group. Particularly, when the artificial nail composition of the invention includes Component B, the artificial nail composition can be suitably used as a photocurable artificial nail composition by including Component C.

Furthermore, when the artificial nail composition of the invention includes Component A and/or Component C, and when Component B has a radically polymerizable group, it is preferable that the artificial nail composition further includes (Component D) a photopolymerization initiator that will be described below. By including Component D, the artificial nail composition of the invention can be more suitably used as a photocurable artificial nail composition.

Component C may be a radically polymerizable compound, or may be a cationically polymerizable compound; however, it is preferable that Component C is a radically polymerizable compound.

Furthermore, Component C is preferably an ethylenically unsaturated compound.

The radically polymerizable compound is a compound having an ethylenically unsaturated bond that is radically polymerizable, and any compound may be used as long as it is a compound having at least one radically polymerizable, ethylenically unsaturated bond in the molecule. This compound includes chemical forms such as a monomer and an oligomer. Regarding the radically polymerizable compound, only one kind may be used, or two or more kinds thereof may be used in combination at any arbitrary ratio, in order to enhance intended characteristics. Furthermore, a polyfunctional compound having two or more functional groups is preferred, rather than a monofunctional compound. Also, it is more preferable to use two or more kinds of polyfunctional compounds in combination, from the viewpoint of controlling the performances such as reactivity and physical properties.

Furthermore, Component C is a compound other than Component A or Component B, and is preferably a compound having a molecular weight of less than 3,000, and more preferably a compound having a molecular weight of less than 1,000.

Examples of the polymerizable compound having a radically polymerizable, ethylenically unsaturated bond include esters and amides of unsaturated carboxylic acids such as acrylic acid, methacrylic acid, itaconic acid, crotonic acid, isocrotonic acid, and maleic acid; anhydrides having ethylenically unsaturated groups; acrylonitrile; styrene; and various radically polymerizable compounds such as unsaturated polyesters, unsaturated polyethers, unsaturated polyamides, and unsaturated urethanes.

Specific examples include (meth)acrylic acid derivatives such as 2-ethylhexyl (meth)acrylate, 2-hydroxyethyl (meth)acrylate, butoxyethyl (meth)acrylate, carbitol (meth)acrylate, cyclohexyl (meth)acrylate, benzyl (meth)acrylate, methyl (meth)acrylate, ethyl (meth)acrylate, n-propyl (meth)acrylate, isopropyl (meth)acrylate, n-butyl (meth)acrylate, allyl (meth)acrylate, glycidyl (meth)acrylate, N-methylol (meth)acrylamide, diacetone (meth)acrylamide, and epoxy (meth)acrylate; and derivatives of allyl compounds such as allyl glycidyl ether, diallyl phthalate, and triallyl trimellitate. More specific examples that can be used include the commercially available products described in Yamashita, Shinzo, ed., "Kakyozai Handobukku (Handbook for Crosslinking Agents)", (1981, Taiseisha, Ltd.); Kato, Kiyomi, ed., "UV-EB Koka Handobukku (Handbook for UV-EB Curing) (Raw Materials Part)" (1985, Kobunshi Kankokai); Radtech Japan, ed., "UV-EB Koka Gijutsu no Oyo to Shijo (Application and Market for UV-EB Curing Technologies)", p. 79 (1989, CMC Publishing Co., Ltd.); Takiyama, Eiichiro, "Poriesuteru Jushi Handobukku (Handbook for Polyesters)", (1988, Nikkan Kogyo Shimbun, Ltd.), and the like; and radically polymerizable or crosslinkable monomers and oligomers that are known in the art.

Regarding Component C, from the viewpoints of removability, adhesiveness and gloss of the artificial nail, it is preferable that Component C includes an ethylenically unsaturated compound having a hydroxyl group, or an ethylenically unsaturated compound having a (poly)alkyleneoxy group; it is more preferable that Component C includes a (meth)acrylate compound having a hydroxyl group, or a (meth)acrylate compound having a (poly)alkyleneoxy group; it is even more preferable that Component C includes a (meth)acrylate compound having a hydroxyl group; and it is particularly preferable that Component C includes a monofunctional (meth)acrylate compound having a hydroxyl group.

Preferred examples of the (meth)acrylate compound having a hydroxy group include hydroxyalkyl (meth)acrylates such as 2-hydroxyethyl (meth)acrylate, 2-hydroxypropyl (meth)acrylate, 3-hydroxypropyl (meth)acrylate, 2,3-dihydroxypropyl (meth)acrylate, and 4-hydroxybutyl (meth)acrylate; polyethylene glycol mono(meth)acrylate, polypropylene glycol mono(meth)acrylate, poly(ethylene glycol-propylene glycol) mono(meth)acrylate, polyethylene glycol-polypropylene glycol mono(meth)acrylate, poly(ethylene glycol-tetramethylene glycol) mono(meth)acrylate, poly(propylene glycol-tetramethylene glycol) mono(meth)acrylate, and polypropylene glycol-polybutylene glycol mono(meth)acrylate.

Preferred examples of the (meth)acrylate compound having a (poly)alkyleneoxy group include methoxypolyethylene glycol (meth)acrylate, octoxypolyethylene glycol-polypropylene glycol (meth)acrylate, lauroxypolyethylene glycol (meth)acrylate, stearoxypolyethylene glycol (meth)acrylate, phenoxypolyethylene glycol (meth)acrylate, phenoxypolyethylene glycol-polypropylene glycol (meth)acrylate, nonylphenoxy-polyethylene glycol (meth)acrylate, nonylphenoxy-polypropylene glycol (meth)acrylate, and nonylphenoxy-poly(ethylene glycol-propylene glycol) (meth)acrylate.

Regarding the (meth)acrylate compound having a hydroxyl group and the (meth)acrylate compound having a (poly)alkyleneoxy group, commercially available products can be used. Representative examples thereof include BLENMER E, BLENMER PE-90, BLENMER PE-200, BLENMER PE-350, BLENMER P, BLENMER PP-1000, BLENMER PP-500, BLENMER PP-800, BLENMER 50PEP-300, BLENMER 70PEP-350B, BLENMER 55PET-800, BLENMER PPT series, BLENMER 10PPB-500B, BLENMER AE-90, BLENMER AE-200, BLENMER AE-400, BLENMER AP-150, BLENMER AP-400, BLENMER AP-550, BLENMER PME-100, BLENMER PME-200, BLENMER PME-400, BLENMER PME-1000, BLENMER 50POEP-800B, BLENMER PLE-200, BLENMER PSE-400, BLENMER PSE-1300, BLENMER PAE-50, BLENMER PAE-100, BLENMER 43PAPE-600B, BLENMER AME-400, BLENMER ALE series, BLENMER ANP-300, BLENMER 75ANP-600, BLENMER AAE-50, and BLENMER AAE-300 (all manufactured by NOF Corp.).

In a case where an ethylenically unsaturated compound having a hydroxyl group is used, the number of hydroxyl groups in the relevant compound is preferably 1 to 10, more preferably 1 to 5, even more preferably 1 to 3, and particularly preferably 1.

Furthermore, in a case where an ethylenically unsaturated compound having a (poly)alkyleneoxy group is used, the repeating unit number of alkyleneoxy groups in the relevant compound is preferably 1 to 25, more preferably 1 to 15, and even more preferably 1 to 10.

Among these, it is preferable that Component C includes a hydroxyalkyl (meth)acrylate compound; it is more preferable that Component C includes 2-hydroxyethyl (meth)acrylate, 2-hydroxypropyl (meth)acrylate, or 3-hydroxypropyl (meth)acrylate; and it is even more preferable that Component C includes 2-hydroxyethyl (meth)acrylate.

Component C may include a polyfunctional polymerizable compound, from the viewpoint of water resistance of the resulting artificial nail.

Examples of the polyfunctional polymerizable compound include a polyfunctional (meth)acrylate compound and a polyol (meth)acrylate compound, all of which have a urethane bond.

Examples OF the polyfunctional (meth)acrylate compound having a urethane bond include preferably a urethane-based addition polymerizable compound that is produced using an addition reaction between an isocyanate group and a hydroxyl group. A specific example thereof is a urethane compound containing two or more ethylenically unsaturated groups in one molecule, which is obtainable by adding an ethylenically unsaturated monomer containing a hydroxyl group to an isocyanate compound having two or more isocyanate groups in one molecule.

Among them, preferred examples of the isocyanate compound include hexamethylene diisocyanate, 2,2,4-trimethylhexamethylene diisocyanate, 2,4,4-trimethylhexamethylene diisocyanate, isophorone diisocyanate, methylenebisphenyl isocyanate, tolylene diisocyanate, m-xylylene diisocyanate, 1,3-bis(isocyanatomethyl)cyclohexane, and DESMODUR (registered trademark) manufactured by Bayer AG.

Specific examples of the ethylenically unsaturated monomer containing a hydroxyl group include 2-hydroxyethyl (meth)acrylate, 2-hydroxypropyl (meth)acrylate, 3-hydroxypropyl (meth)acrylate, 2,3-dihydroxypropyl (meth)acrylate, 4-hydroxybutyl (meth)acrylate, polyethylene glycol mono(meth)acrylate, polypropylene glycol mono(meth)acrylate, poly(ethylene glycol-propylene glycol) mono(meth)acrylate, polyethylene glycol-polypropylene glycol mono(meth)acrylate, poly(ethylene glycol-tetramethylene glycol) mono(meth)acrylate, poly(propylene glycol-tetramethylene glycol) mono(meth)acrylate, polypropylene glycol-polybutylene glycol mono(meth)acrylate, glycerin di(meth)acrylate, pentaerythritol tri(meth)acrylate, and dipentaerythritol penta(meth)acrylate.

A preferred example of the polyol (meth)acrylate compound is a (meth)acrylic acid ester of a bifunctional or higher-functional polyol compound. Specific preferred examples thereof include glycerin di(meth)acrylate, pentaerythritol tri(meth)acrylate, dipentaerythritol penta(meth)acrylate, ethylene glycol di(meth)acrylate, pentaerythritol tetra(meth)acrylate, dipentaerythritol hexa(meth)acrylate, polyethylene glycol di(meth)acrylate, propylene glycol di(meth)acrylate, polypropylene glycol di(meth)acrylate, 1,4-butanediol di(meth)acrylate, trimethylolpropane tri(meth)acrylate, ditrimethylolpropane tetra(meth)acrylate, tris(2-(meth)acryloyloxyethyl)isocyanuric acid, and 2,2-bis(4-(2-(meth)acryloyloxyethyl)oxyphenyl)propane.

Component C may be included singly, or two or more kinds thereof may be used in combination.

The content of Component C in the artificial nail composition of the invention is not particularly limited; however, the content of Component C is preferably 1% to 80% by mass, more preferably 3% to 70% by mass, even more preferably 10% to 60% by mass, and particularly preferably 20% to 50% by mass, relative to the total mass of the artificial nail composition, as the total amount of polymerizable compounds including Component A. When the content is in the range described above, the resulting artificial nail has superior removability and water-resistance.

### (Component D) Photopolymerization initiator

It is preferable that the artificial nail composition of the invention includes (Component D) a photopolymerization initiator. When the artificial nail composition includes Component D, the artificial nail composition of the invention can be suitably used as a photocurable artificial nail composition.

Examples of the photopolymerization initiator include a radical photopolymerization initiator and a cationic photopolymerization initiator; however, it is more preferable that the artificial nail composition includes a radical photopolymerization initiator.

Regarding the photopolymerization initiator, any known photopolymerization initiator can be used. The photopolymerization initiator that can be used in the invention may be used singly, or may be used in combination of two or more kinds thereof. Furthermore, a radical photopolymerization initiator and a cationic photopolymerization initiator may be used together.

The photopolymerization initiator that can be used for the invention is a compound which absorbs light and produces a polymerization initiator species. Examples of the light include γ-radiation, β-radiation, an electron beam, ultraviolet radiation, visible light, and infrared radiation. Among these, ultraviolet radiation is suitably used.

Specific preferred examples of the photopolymerization initiator that can be used for the invention include the following compounds. However, the invention is not intended to be limited to these examples:

an acetophenone derivative (for example, 1-hydroxycyclohexyl phenyl ketone, acetophenone, 2,2-dimethoxy-2-phenylacetophenone, 2,2-diethoxyacetophenone, 2-hydroxy-2-methylpropiophenone, 4'-isopropyl-2-hydroxy-2-methylpropiophenone, 2-methyl-1-[4-(methylthio)phenyl]-2-morpholino-1-propane, 2-benzyl-2-dimethylamino-1-(4-morpholinophenyl)-butan-1-one, benzoin, benzoin methyl ether, benzoin ethyl ether, or benzoin propyl ether);
a benzophenone derivative (for example, benzophenone, 4,4'-bis(dimethylamino)benzophenone, or 3,3-dimethyl-4-methoxybenzophenone);
an anthraquinone derivative (for example, anthraquinone, 2-methylanthraquinone, 2-ethylanthraquinone, or tert-butylanthraquinone);
a thioxanthone derivative (for example, 2-chlorothioxanthone, diethylthioxanthone, isopropylthioxanthone, or diisopropylthioxanthone);
a trihaloalkyl compound (for example, 2,4,6-(trichloromethyl)triazine, 2,4-trichloromethyl-6-(4-methoxyphenyl)triazine, or tribromomethylphenylsulfone);
a lophine dimer derivative (for example, 2-(o-chlorophenyl)-4,5-diphenylimidazolyl dimer);
an acridine derivative (for example, 9-phenylacridine, 1,7-bis(9-acridinyl)heptane, 1,5-bis(9-acridinyl)pentane, or 1,3-bis(9-acridinyl)pronopane);
a phsophine oxide derivative (for example, trimethylbenzoyldiphenylphosphine oxide or bis(2,4,6-trimethylbenzoyl)phenylphosphine oxide);
a metallocene derivative (for example, biscyclopentadienylbis(difluoropyrrylphenyl)titanium);
an onium salt (for example, bis(4-t-butylphenyl)iodonium tosylate or triphenylsulfonium tosylate);

an oxime ester derivative (for example, 1-(4-phenylthiophenyl)-1,2-octanedione-2-(O-benzoyloxime) or 1-[9-ethyl-6-(2-methylbenzoyl)-9H-carbazol-3-yl]-ethanone-1-(O-acetyloxime));
a borate salt (for example, tetraphenylborate sodium salt); and
a peroxide (for example, t-butyl perbenzoate).

The photopolymerization initiator used in the invention is appropriately selected in order to impart photocurability and photoselectivity of the artificial nail composition; however, among these, Component D is preferably a photopolymerization initiator selected from the group consisting of an acetophenone derivative, a benzophenone derivative, a lophine dimer derivative, a phosphine oxide derivative, a metallocene derivative, an oxime ester derivative, an onium salt, and a borate salt; and more preferably a photopolymerization initiator selected from the group consisting of an acetophenone derivative, a benzophenone derivative, a phosphine oxide derivative, a metallocene derivative, and an oxime ester derivative.

The content of Component D in the artificial nail composition of the invention is preferably 0.01% to 20% by mass, more preferably 0.1% to 15% by mass, and even more preferably 0.5 to 12% by mass, relative to the total mass of the artificial nail composition. When the content of Component D is in the range described above, the resulting artificial nail has superior adhesiveness, gloss and water resistance.

### (Component E) Solvent

The artificial nail composition of the invention may include (Component E) a solvent, from the viewpoint of coatability.

Regarding the solvent, any known solvents can all be suitably used. Examples thereof include alcohol-based solvents such as ethanol, isopropanol, ethylene glycol monomethyl ether, ethylene glycol monobutyl ether, propylene glycol monomethyl ether, diethylene glycol monoethyl ether, and dipropylene glycol monomethyl ether, and solvents based on acetates thereof; ester-based solvents such as ethyl acetate and butyl acetate; ketone-based solvents such as acetone, methyl ethyl ketone, methyl isobutyl ketone, and cyclohexanone; ether-based solvents such as tetrahydrofuran and methyl t-butyl ether; and water. However, the solvent is not intended to be limited to these examples.

From the viewpoint of the drying speed, the boiling point of the solvent is preferably 30°C to 130°C, more preferably 35°C to 100°C, even more preferably 40°C to 90°C, and particularly preferably 50°C to 85°C. When the boiling point is in the range described above, superior dryability and coatability are obtained.

Component E may be included singly, or two or more kinds thereof may be used together.

It is preferable that Component E includes an alcohol-based solvent and/or a ketone-based solvent, and it is more preferable that Component E includes an alcohol-based solvent and a ketone-based solvent.

The content of Component E in the artificial nail composition of the invention is not particularly limited; however, the content is preferably 1% to 90% by mass, more preferably 2% to 80% by mass, even more preferably 3% to 80% by mass, and particularly preferably 5% to 50% by mass, relative to the total mass of the artificial nail composition. When the content of Component E is in the range described above, superior coatability is obtained, and the resulting artificial nail has superior removability and adhesiveness.

### <Other components>

In the artificial nail composition of the invention, additive components that can be commonly incorporated into an artificial nail composition as additional components other than the Component A to Component E described above, can be incorporated to the extent that the effects of the invention are not impaired. Examples of such additive components include an anti-foaming agent, a buffering agent, a chelating agent, a dispersant, a dye, a filler, a pigment, an antiseptic, a resin powder (for example, poly(meth)acrylic acid), an inorganic powder (for example, silica gel), a thickener, a wetting agent, and a radical polymerization inhibitor (polymerization inhibitor). However, the additive components are not intended to be limited to these.

Furthermore, the amounts of incorporation of the various components in the artificial nail composition of the invention are not particularly limited; however, it is preferable that the mass ratio of Component A and Component C/Component B and other polymers/Component D/other components is in the range of 0 to 70/10 to 90/0 to 25/0 to 20. When the mass ratio is in the range described above, superior coatability is obtained, and the resulting artificial nail has superior removability and gloss.

In a case where the artificial nail composition of the invention is used as an artificial nail composition for a manicure liquid, it is preferable that the artificial nail composition includes at least Component B and Component E, and in addition to these, a polymer other than Component B, and other additive components that can be commonly incorporated into an artificial nail composition can be incorporated to the artificial nail composition.

Furthermore, in a case where the artificial nail composition of the invention is used as a photocurable artificial nail composition (artificial nail composition for gel nail), it is preferable that the artificial nail composition includes at least Component A and Component D, or the artificial nail composition includes Component B, Component A and/or Component C, and Component D. Furthermore, when the artificial nail composition includes Component A and Component D, it is preferable that the nail composition includes Component B and/or a polymer other than Component B. In addition to the components described above, Component E, and other additive components that can be commonly incorporated into an artificial nail composition can be arbitrarily incorporated into the artificial nail composition.

The acid value of the artificial nail composition of the invention is preferably 0.5 mmol/g to 7.0 mmol/g, more preferably 0.75 mmol/g to 6.5 mmol/g, and even more preferably 1.0 mmol/g to 6.0 mmol/g. When the acid value of the artificial nail composition is in the range described above, removability and long-lastingness are improved.

It is preferable to appropriately adjust the contents of Component A and/or Component B such that the acid value is adjusted to the range described above.

### (Artificial nail)

The artificial nail of the invention is an artificial nail having a layer formed from the artificial nail composition of the invention, and it is preferable that the artificial nail of the invention is an artificial nail having a layer formed by drying and/or exposing to light the artificial nail composition of the invention.

The artificial nail of the invention is suitable as a nail polish such as for a manicure or pedicure, or as a gel nail.

The artificial nail of the invention may have at least a portion thereof formed from the artificial nail composition of the invention, and may have another layer or structure, or may have the entire artificial nail formed from the artificial nail composition of the invention.

The layer formed from the artificial nail composition of the invention can be suitably used as any of a primer layer, a base layer, a color layer, and/or a top layer in the artificial nail of the invention.

Furthermore, the artificial nail of the invention may have only one layer of the layer formed from the artificial nail composition of the invention, or may have two or more such layers.

Above all, from the viewpoint of removability, it is preferable that at least one layer formed from the nail composition of the invention is a layer that is in contact with the nail.

According to the invention, when it is intended to form an artificial nail using a photocurable artificial nail composition, the artificial nail can be formed by first applying the artificial nail composition of the invention on or above the nail (on or above the nail of a human being or an animal, or on or above a support), subsequently irradiating the artificial nail composition with ultraviolet radiation, and thereby photocuring the artificial nail composition of the invention.

Preferably, the artificial nail can be formed by first forming a base layer by applying the artificial nail composition of the invention on the nail, subsequently irradiating the artificial nail composition with ultraviolet radiation, and thereby photocuring the artificial nail composition of the invention; subsequently forming a color layer thereon by applying a known artificial nail composition containing a colorant, or the artificial nail composition of the invention containing a colorant, and photocuring the artificial nail composition; and lastly forming a top layer thereon by applying a known artificial nail composition or the artificial nail composition of the invention.

Fig. 1 illustrates a more preferred configuration of the artificial nail according to the invention.

According to the invention, more preferably, the artificial nail can be formed by first forming a base layer 2 by applying the artificial nail composition of the invention on the nail 1, subsequently irradiating the artificial nail composition with ultraviolet radiation, and thereby photocuring the artificial nail composition of the invention; subsequently forming a color layer 3 by applying a known artificial nail composition containing a colorant thereon, and photocuring the artificial nail composition; and lastly forming a top layer 4 by applying a known artificial nail composition thereon. When an artificial nail is formed in this manner, stability of the artificial nail can be enhanced. Furthermore, more preferably, the artificial nail can be formed by forming a base layer 2 by applying the artificial nail composition of the invention on the nail 1, subsequently irradiating the artificial nail composition with ultraviolet radiation, and thereby photocuring the artificial nail composition of the invention; subsequently forming a color layer 3 by applying the artificial nail composition of the invention containing a colorant thereon, and photocuring the artificial nail composition; and lastly forming a top layer 4 by applying the artificial nail composition of the invention thereon. When an artificial nail is formed in this manner, removability of the artificial nail can be further enhanced.

Furthermore, there are no particular limitations on the thickness of a layer formed from the artificial nail composition of the invention in the artificial nail of the invention; however, the thickness is preferably 10 to 2,000 µm, more preferably 20 to 1,500 µm, and even more preferably 20 to 400 µm.

### (Artificial nail forming method)

The artificial nail forming method of the invention is not particularly limited as long as it is a method of forming an artificial nail using the artificial nail composition of the invention; however, the method is preferably a method including a step of applying the artificial nail composition of the invention on or above the nail of a human being or an animal, or on or above another artificial nail to form a coating film; and a step of drying and/or exposing to light the coating film, and thus forming an artificial nail.

In order to form an artificial nail from the artificial nail composition of the invention, a forming method based on any of drying or light exposure can be suitably selected.

When the artificial nail composition of the invention includes Component A, it is preferable that the artificial nail composition includes Component B and/or a polymer other than Component B, and (Component D) a photopolymerization initiator, and the artificial nail composition may optionally include (Component E) a solvent.

Furthermore, when the artificial composition of the invention includes Component B, it is preferable that the artificial nail composition includes at least Component A and/or Component C, and Component D, or the composition includes at least Component E.

Furthermore, there are no particular limitations on the thickness of a layer formed from the artificial nail composition of the invention according to the artificial nail forming method of the invention; however, the thickness is preferably 10 to 2,000 µm, more preferably 20 to 1,500 µm, and even more preferably 20 to 400 µm.

### <Application step>

It is preferable that the artificial nail forming method of the invention includes a step of applying the artificial nail composition of the invention on or above the nail of a human being or an animal, or on or above another artificial nail, to form a coating film (application step).

The other artificial nail is not particularly limited as long as it is a substrate used for an artificial nail, and examples thereof include a resin substrate, an artificial nail formed by a method other than the artificial nail forming method of the invention, and an artificial nail obtained by the artificial nail forming method of the invention.

There are no particular limitations on the application method, and application may be carried out by a known method; however, a method of applying the artificial nail composition using a painting brush, an ink brush or the like may be preferably used. Furthermore, a spray application or inkjet application may also be implemented.

The thickness of the coating film is also not particularly limited, and the coating thickness may be appropriately adjusted in consideration of the desired thickness for the resulting artificial nail.

### <Drying or exposure step>

It is preferable that the artificial nail forming method of the invention includes a step of drying and/or exposing to light the coating film, and thus forming an artificial nail (drying or exposure step).

### - Drying step -

In a case where an artificial nail is formed by drying, it is preferable that the artificial nail composition of the invention used includes (Component E) a solvent.

The drying method is not particularly limited, and drying may be carried out by any known method.

Specifically, preferred examples include a method of drying the artificial nail composition by leaving the artificial nail composition to stand at room temperature (for example, 10°C to 30°C); a method of drying the artificial nail composition by leaving the artificial nail composition to stand under a gas stream; a method of heating and drying the artificial nail composition; and methods combining these. There are no particular limitations on the heating method; however, examples thereof include a method of leaving the artificial nail composition to stand in an atmosphere at a temperature higher than room temperature; a method of leaving the artificial nail composition to stand under a heating gas stream; and a method of heating the artificial nail composition using a heating means such as a heater or an infrared lamp (IR lamp).

There are no particular limitations on the drying time, and the drying time may be appropriately adjusted depending on the composition of the artificial nail composition, the drying method, and the coating thickness. However, from the viewpoint of convenience and from the viewpoint of imparting smoothness by self leveling or glossiness, the drying time is preferably 10 seconds to 20 minutes, more preferably 15 seconds to 10 minutes, even more preferably 30 seconds to 5 minutes, and particularly preferably 30 seconds to 2 minutes.

### - Exposure step -

In a case where an artificial nail is formed by exposure to light, it is preferable that the artificial nail composition of the invention used includes (Component A) a compound having an ethylenically unsaturated group and an acid group, and/or (Component C) another polymerizable compound, and (Component D) a photopolymerization initiator.

Furthermore, in a case where the artificial nail composition of the invention includes (Component E) a solvent in addition to these components, it is preferable to perform drying before performing exposure to light.

Examples of the light used for exposure include ultraviolet radiation and visible light, and from the viewpoints of curing sensitivity and easy availability of apparatuses, ultraviolet radiation is particularly preferred.

There are no particular limitations on the exposure means, and any known exposure means can be used; however, examples thereof include ultraviolet lamps (UV lamps) such as a mercury lamp and a metal halide lamp; a light emitting diode (LED); and a laser diode (LD).

Among them, a UV lamp or an ultraviolet light emitting diode (UV-LED) is preferred.

There are no particular limitations on the exposure time; however, the exposure time is preferably 0.5 to 15 minutes, more preferably 0.5 to 10 minutes, and even more preferably 0.5 to 7 minutes. Furthermore, exposure to light may be performed intermittently or may be performed continuously, or may be performed using pulsed light, and exposure may be achieved by any arbitrary method.

It is preferable that the artificial nail forming method of the invention further includes, after the drying or exposure step, a step of cleaning or wiping the surface of the artificial nail thus obtained, from the viewpoint of gloss or cosmetic appearance of the resulting artificial nail, and from the viewpoint of removing uncured components in the case of performing exposure to light.

Examples of the method of cleaning or wiping include a method of wiping the artificial nail using a wiping substrate such as a wipe sheet or a sponge wipe, which has been made to include a solvent such as ethanol; and a method of cleaning using a solvent such as ethanol, or water.

Furthermore, it is preferable that the solvent used for the cleaning or wiping is a solvent which does not dissolve the artificial nail thus obtained.

Also, it is preferable that the artificial nail forming method of the invention includes, in the case of performing exposure to light, a step of roughening the surface of the nail of a human being or an animal, or the surface of another artificial nail, before the application step. According to this embodiment, the artificial nail obtained by exposing the artificial nail composition to light (gel nail) has excellent adhesiveness and durability.

The method for roughening the nail surface is not particularly limited, and roughening may be carried out by any known method. For example, a method of performing roughening using a nail file such as a file may be preferably used.

Furthermore, the artificial nail forming method of the invention may include any other known processes.

### (Artificial nail removal method)

The artificial nail removal method of the invention includes a step of removing the artificial nail of the invention by bringing the artificial nail into contact with a removal liquid (removal step).

The removal liquid is an aqueous solution having a pH of from 8 to 11.

The artificial nail composition of the invention can also be removed with an organic solvent such as acetone, which is a conventional removal liquid; however, it is possible to remove the artificial nail composition more safely using an aqueous solution having a pH of from 8 to 11, without imposing a burden on the skin and the nail. It is preferable that the removal liquid that is suitably used in the invention includes an organic/inorganic acid component for basifying the nail, water, an optional stabilizer, and an optional surfactant. A preferred embodiment of the removal liquid is described below.

According to the invention, for the removal liquid, an aqueous solution having any composition can be suitably used as long as the aqueous solution has a pH of from 8 to 11; however, it is preferable that the removal liquid includes an alkali agent for maintaining the pH of the removal liquid at 8 to 11 (pH buffering agent) as an essential component, and includes a surfactant for enhancing removability.

### <Alkali agent (pH buffering agent)>

Regarding the alkali agent for maintaining the pH of the removal liquid at 8 to 11 (pH buffering agent), any known compound can be used; however, preferred examples include carbonates (sodium carbonate, potassium carbonate, and the like), hydrogen carbonates (sodium hydrogen carbonate, potassium hydrogen carbonate, and the like), hydroxide salt (sodium hydroxide, potassium hydroxide, and the like), phosphates (trisodium phosphate, and the like), borates (potassium borate, and the like), and organic amine compounds (triethylamine, triethanolamine, and the like), and particularly preferred examples include carbonates and hydrogen carbonates. Furthermore, these alkali agents may be used as mixtures, and it is particularly preferable to use a carbonate and a hydrogen carbonate in combination.

The percentage content of the alkali agent in the removal liquid used for the invention is preferably 1% to 20% by mass, more preferably 3% to 15% by mass, and particularly preferably 4% to 12% by mass, relative to the mass of the removal liquid. When the total amount is 1% by mass or more, removability is not decreased, and when the total amount is 20% by mass or less, precipitates or crystals of the alkali agent and the like are not easily produced.

### <Surfactant>

Regarding the surfactant, known surfactants such as an anionic surfactant, a cationic surfactant, a neutral surfactant, and an amphoteric surfactant can be used.

Examples of the anionic surfactant include carboxylic acid salts (for example, sodium linolate and potassium oleate), sulfonic acid salts (for example, alkanesulfonic acid salts, dialkylsulfosuccinic acid salts, linear alkylbenzenesulfonic acid salts, and alkylnaphthalenesulfonic acid salts), sulfuric acid monoester salts (for example, alkylsulfuric acid ester salts), and phosphoric acid ester salts (for example, alkylphosphoric acid ester salts). Among these, sulfuric acid salts and carboxylic acid salts are preferred; sulfonic acid salts are more preferred; and dialkylsulfosuccinic acid salts, alkylsulfuric acid ester salts, alkylbenzenesulfonic acid salts, and alkylnaphthalenesulfonic acid salts are particularly preferably used.

The cationic surfactant is not particularly limited, and any conventionally known cationic surfactant can be used. Examples thereof include alkylamine salts, quaternary ammonium salts, polyoxyethylene alkylamine salts, and polyethylene polyamine derivatives.

Examples of the nonionic surfactant include a polyethylene glycol type higher-alcohol ethylene oxide adduct, an alkylphenol ethylene oxide adduct, a fatty acid ethylene oxide adduct, a poly alcohol fatty acid ester ethylene oxide adduct, a higher alkylamine ethylene oxide adduct, a fatty acid amide ethylene oxide adduct, an ethylene oxide adduct of oils and fats, a polypropylene glycol ethylene oxide adduct, a dimethylsiloxane-ethylene oxide block copolymer, a dimethylsiloxane-(propyleneoxide-ethylene oxide) block copolymer, a fatty acid ester of poly alcohol type glycerol, a fatty acid ester of pentaerythritol, fatty acid esters of sorbitol and sorbitan; a fatty acid ester of sucrose, an alkyl ether of a poly alcohol, and a fatty acid amide of an alkanolamine.

According to the invention, an ethylene oxide adduct of a sorbitol and/or sorbitan fatty acid ester, a polypropylene glycol ethylene oxide adduct, a dimethylsiloxane-ethylene oxide block copolymer, a dimethylsiloxane-(propylene oxide-ethylene oxide) block copolymer, and a fatty acid ester of a poly alcohol are more preferred.

Furthermore, from the viewpoint of stable solubility in water or turbidity, the nonionic surfactant is preferably a nonioinc surfactant having a Hydrophile-Lipophile Balance (HLB) value of 6 or more, and more preferably 8 or more. Furthermore, acetylene glycol-based and acetylene alcohol-based oxyethylene adducts, and fluorine-based and silicon-based surfactants can also be used similarly.

The amphoteric surfactant is a compound having an anionic moiety and a cationic moiety in the same molecule, as is well known in the field of surfactants, and amphoteric surfactants such as amino acid-based, betaine-based, and amine oxide-based surfactants are included in the class. The amphoteric surfactant used in the removal liquid that is used in the invention is preferably a compound represented by the following Formula <1> and a compound represented by the following Formula <2>.

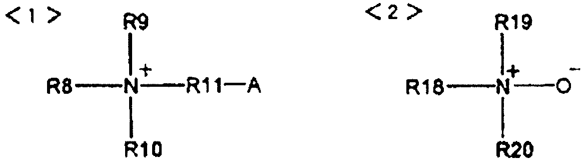

In Formula <1>, R8 represents an alkyl group; R9 and R10 each represent a hydrogen atom or an alkyl group; R11 represents an alkylene group; and A represents a carboxylic acid ion or a sulfonic acid ion.

In Formula <2>, R18, R19, and R20 each represent a hydrogen atom or an alkyl group; however, all of R18, R19 and R20 are not necessarily hydrogen atoms.

In regard to Formula <1>, the alkyl group represented by R8, R9 or R10 and the alkylene group represented by R11 may be linear or branched, may also have a linking group in the chain, and may have a substituent. It is preferable that the alkyl group and the alkylene group contain a heteroatom such as an ester bond, an amide bond, or an ether bond, as the linking group. Furthermore, the substituent is preferably a hydroxyl group, an alkoxy group having 1 to 8 carbon atoms, a phenyl group, an amide group, a halogen atom, or the like. Furthermore, R8, R9 or R10 may be linked to one another in a cyclic form. Regarding the form of linkage in a cyclic form, it is preferable that the substituents form a 5-membered ring or a 6-membered ring, and it is particularly preferable that the ring is a heterocyclic ring containing a heteroatom. The heteroatom is preferably an oxygen atom, a nitrogen atom, or a sulfur atom. Particularly, it is preferable that the ring has a cationic structure such as a substituted imidazole ring, a substituted imidazoline ring, or a substituted imidazolidine.

In regard to the compound represented by Formula <1>, when the total carbon number value increases, the hydrophobic moiety becomes larger, and dissolution of the compound in a water-based removal liquid becomes difficult. In this case, dissolution is ameliorated by adding an organic solvent, for example, a dissolution aid such as an alcohol; however, if the total carbon number value is too large, surfactants may not be dissolved in an appropriate range of incorporation. Therefore, the sum of the carbon numbers of R8 to R11 is preferably 8 to 25, and more preferably 11 to 21.

In regard to Formula <2>, the alkyl group represented by R18, R19 or R20 may be linear or branched, may have a linking group in the chain, and may have a substituent. It is preferable that the alkyl group contains a heteroatom such as an ester bond, an amide bond, or an ether bond, as the linking group. Furthermore, the substituent is preferably a hydroxyl group, an alkoxy group having 1 to 8 carbon atoms, a phenyl group, an amide group, a halogen atom, or the like.

In regard to the compound represented by Formula <2>, when the total carbon number value increases, the hydrophobic moiety becomes larger, and dissolution of the compound in a water-based removal liquid becomes difficult. In this case, dissolution is ameliorated by adding an organic solvent, for example, a dissolution aid such as an alcohol; however, if the total carbon number value is too large, surfactants may not be dissolved in an appropriate range of incorporation. Therefore, the sum of the carbon numbers of R18 to R20 is preferably 8 to 22, and more preferably 10 to 20.

These surfactants are appropriately selected in accordance with the materials used in the artificial nail composition; however, an anionic surfactant, a nonionic surfactant, and an amphoteric surfactant are preferred, and an anionic surfactant and an amphoteric surfactant are particularly preferred. Furthermore, two or more kinds of surfactants can be used in combination, and in the case of using surfactants in combination, a combination of anionic surfactants, a combination of amphoteric surfactants, a combination of an anionic surfactant and a nonionic surfactant, and a combination of an anionic surfactant and a nonionic surfactant are preferred.

The content of the surfactant in the removal liquid is preferably 0.01% to 10% by mass, and more preferably 0.02% to 9% by mass.

### <Other components>

The removal liquid used in the invention may include, in addition to the alkali agent and the surfactant, a wetting agent, an antiseptic, a chelate compound, an anti-foaming agent, an organic acid, an organic solvent, an inorganic acid, an inorganic salt, a water-soluble polymer compound, a fragrance, and the like.

Examples of the wetting agent that are suitably used include ethylene glycol, propylene glycol, triethylene glycol, butylene glycol, hexylene glycol, diethylene glycol, dipropylene glycol, glycerin, trimethylolpropane, and diglycerin. The wetting agent may be used singly, or may be used in combination of two or more kinds thereof. Generally, a wetting agent is used in an amount of 0% to 5% by mass based on the total mass of the removal liquid.

Examples of the antiseptic that can be preferably used include phenol or a derivative thereof, formalin, an imidazole derivative, sodium dehydroacetate, 4-isothiazolin-3-one derivative, benzisothiazolin-3-one, 2-methyl-4-isothiazolin-3-one, a benzotriazole derivative, an amidineguanidine derivative, a quaternary ammonium salt, derivatives of pyridine, quinoline, guanidine and the like, diazine, a triazole derivative, oxazole, an oxazine derivative, nitrobromoalcohol-based 2-bromo-2-nitropropane-1,3-diol, 1,1-dibromo-1 -nitro-2-ethanol, and 1,1-dibromo-1-nitro-2-propanol. It is preferable to use two or more kinds of antiseptics in combination so that the artificial nail composition is efficacious against various fungi and disinfection. The amount of addition of the antiseptic is an amount which stably exhibits efficacy against bacteria, fungi, yeast and the like, and may vary depending on the kind of bacteria, fungi and yeast; however, the amount of addition is preferably in the range of 0% to 4% by mass based on the removal liquid.

Examples of the chelate compound include ethylenediaminetetraacetic acid, potassium salt thereof, and sodium salt thereof; diethylenetriaminepentaacetic acid, potassium salt thereof, and sodium salt thereof; triethylenetetraminehexaacetic acid, potassium salt thereof, and sodium salt thereof; hydroxyethylethylenediaminetriacetic acid, potassium salt thereof, and sodium salt thereof; nitrilotriacetic acid, potassium salt thereof, and sodium salt thereof; 1-hydroxyethane-1,1-diphosphonic acid, potassium salt thereof, and sodium salt thereof; an organic phosphonic acid or phosphonoalkanetricarboxylic acid, such as aminotri(methylenephosphonic acid), potassium salt thereof, and sodium salt thereof. An organic amine salt is also effectively used instead of sodium salt and potassium salt of a chelating agent described above. The amount of addition is suitably 0% to 1.0% by mass based on the removal liquid.

Regarding the anti-foaming agent, general silicone-based self-emulsifying type, emulsifying type, and nonionic compounds having a HLB value of 5 or less can be used. Among these, silicone anti-foaming agent are preferred, and emulsifying dispersion type and solubilizing type of anti-foaming agents can also be used. The content of the anti-foaming agent is suitably in the range of 0% to 1.0% by mass based on the removal liquid.

Examples of the organic acid include citric acid, acetic acid, oxalic acid, malonic acid, salicylic acid, caprylic acid, tartaric acid, malic acid, lactic acid, levulinic acid, p-toluenesulfonic acid, xylenesulfonic acid, phytic acid, and organic phosphonic acid. The organic acid can also be used in the form of an alkali metal salt or ammonium salt thereof. The content of the organic acid is preferably 0% to 0.5% by mass based on the treatment liquid.

Examples of the organic solvent include aliphatic hydrocarbons (hexane, heptane, "ISOPAR E, H and G" (Esso Chemical, Ltd.), gasoline, kerosene, and the like), aromatic hydrocarbons (toluene, xylene, and the like), halogenated hydrocarbons (methylene dichloride, ethylene dichloride, trichlene, monochlorobenzene, and the like), and polar solvents.

Examples of the polar solvents include alcohols (methanol, ethanol, propanol, isopropanol, propylene glycol monoethyl ether, propylene glycol monomethyl ether, and the like), ketones (acetone, methyl ethyl ketone, and the like), esters (ethyl acetate, propyl acetate, butyl acetate, methyl lactate, ethyl lactate, and the like), and others (triethyl phosphate, tricresyl phosphate, N-phenylethanolamine, N-phenyldiethanolamine, and the like).

Furthermore, in a case where the organic solvent is insoluble in water, it is possible to use the organic solvent after solubilizing the organic solvent in water using a surfactant or the like. According to the invention, it is preferable that the removal liquid has water as a main component, and in a case where the removal liquid contains an organic solvent, the concentration of the solvent is preferably less than 40% by mass from the viewpoints of safety and flammability.

Examples of the inorganic acid and inorganic salt include phosphoric acid, metaphosphoric acid, monobasic ammonium phosphate, dibasic ammonium phosphate, monobasic sodium phosphate, dibasic sodium phosphate, monobasic potassium phosphate, dibasic potassium phosphate, sodium tripolyphosphate, potassium pyrophosphate, sodium hexametaphosphate, magnesium nitrate, sodium nitrate, potassium nitrate, ammonium nitrate, sodium sulfate, potassium sulfate, ammonium sulfate, sodium sulfite, ammonium sulfite, sodium hydrogen sulfate, and nickel sulfate. The content of the inorganic salt is preferably an amount of 0% to 0.5% by mass based on the total mass of the removal liquid.

Examples of the water-soluble polymer compound include soybean polysaccharides, modified starch, gum arabic, dextrin, cellulose derivatives (for example, carboxymethyl cellulose, carboxyethyl cellulose, and methyl cellulose) and modification products thereof, pullulan, polyvinyl alcohol and derivatives thereof, polyvinylpyrrolidone, polyacrylamide and acrylamide copolymers, a vinyl methyl ether/maleic anhydride copolymer, a vinyl acetate/maleic anhydride copolymer, and a styrene/maleic anhydride copolymer. A preferred acid value of the water-soluble polymer compound is 0 to 3.0 meq/g. The water-soluble polymer compound can be used in combination of two or more kinds thereof. The content of the water-soluble polymer compound in the removal liquid is preferably 0% to 20% by mass, and more preferably 0.1% to 10% by mass.

Regarding the fragrance, any known fragrance can be appropriately selected and used, and fragrances can be used singly, or plural fragrances can be used in combination. It is preferable that the fragrance is appropriately selected in accordance with the compounds or solvent used in the removal liquid, and it is preferable to use known fragrances in combination to be optimized. Furthermore, regarding the fragrance, natural fragrances and synthetic fragrances can be used. Regarding the fragrance used in the invention, the fragrances described in JP2009-298109A can be suitably used. The content of the fragrance in the removal liquid is preferably 0% to 20% by mass, and particularly preferably 0.02% to 10% by mass.

### <Water and pH>

The water used in the removal liquid is not particularly limited, and tap water, distilled water, ion-exchanged water or the like can be used.

The content of water in the removal liquid is not particularly limited; however, the content is preferably 50% to 99.5% by mass, more preferably 70% to 99% by mass, and even more preferably 90% to 99% by mass, relative to the total mass of the removal liquid.

The pH of the removal liquid is preferably 8 or higher from the viewpoint of the ability to ionize the acid groups of Component A and Component B included in the artificial nail of the invention, that is, removability, and the pH is preferably 11 or lower from the viewpoint of the influence on the skin or the like. The pH of the removal liquid is preferably 8.5 to 10.9, more preferably 8.7 to 10.8, and even more preferably 9 to 10.7.

It is preferable that the removal liquid is used after being warmed to the extent that does not impair safety, from the viewpoint of accelerating the rate of removal. Warming is preferably carried out at 30°C to 45°C, more preferably 35°C to 45°C, and particularly preferably 40°C to 45°C. Furthermore, in a case where the artificial nail is immersed in the removal liquid, the liquid for immersion may be stirred.

The artificial nail of the invention can be conveniently removed by bringing the artificial nail into contact with an aqueous solution at pH 8 to 11, instead of the conventional organic solvent such as acetone.

The method for contact is not particularly limited, and examples include a method of immersing the artificial nail directly into the removal liquid; a method of mounting cotton that has been soaked with the removal liquid or the like on the artificial nail and leaving the artificial nail to stand; and a method of dropping or spraying the removal liquid using a sprayer or a shower onto the artificial nail. However, the invention is not intended to be limited to these examples.

A specific example of the removal method is described below. That is, the surface of an artificial nail is coarsely scratched by scraping the surface of the artificial nail with a file (nail file), subsequently cotton soaked with a removal liquid is mounted on the artificial nail, this artificial nail and cotton are wrapped with aluminum foil or the like and are left to stand for about 3 to 5 minutes so as to swell the artificial nail, and then this swollen artificial nail can be detached very easily and safely using a spatula-shaped or stick-shaped tool or the like, without imposing a burden on the fingertip or the nail. Furthermore, since an organic solvent is not used, it is also preferable from the viewpoint of environment protection.

Meanwhile, in the method described above, even if the operation of wrapping the cotton that has been soaked with the removal liquid using aluminum foil is not carried out, a removal effect equivalent to the effect described above can be obtained by simply lengthening the time for leaving the cotton to stand after mounting.

Furthermore, the artificial nail can be swollen by immersing the fingertip (in a state of having the artificial nail formed thereon) in a removal liquid (aqueous solution at pH 8 to 11) for about 3 to 5 minutes, instead of using cotton. Thus, the removal can be achieved more conveniently by scraping the artificial nail using a brush or with the hand in the removal liquid.

The contact time is not particularly limited, and may be any length of time in which the artificial nail can be removed; however, the contact time is preferably 0.5 to 10 minutes, and more preferably 1 to 5 minutes.

Furthermore, it is preferable that the artificial nail removal method of the invention includes a step of scratching the surface of the artificial nail so that the layer formed from the artificial nail composition of the invention is partially exposed at the surface layer.

The method of scratching is not particularly limited; however, a nail file such as a file can be suitably used.

In regard to the removal method of the invention, the temperature of the removal liquid (aqueous solution at pH 8 to 11) is preferably 10°C to 50°C, preferably 20°C to 45°C, and particularly preferably 30°C to 45°C. When the temperature is adjusted to the above-mentioned temperature, removability is increased.

A specific preferred example of the removal method is described below. That is, optionally, the artificial nail surface is coarsely scratched by scraping the artificial nail surface using a nail file to the extent that the layer formed from the artificial nail composition of the invention is partially exposed at the surface layer, subsequently the artificial nail is immersed in a removal liquid (aqueous solution at pH 8 to 11), the artificial nail is left to stand in an immersed state for about 1 to 5 minutes to alter the solubility of the artificial nail, and then the artificial nail can be detached very easily and safely by wiping the artificial nail with a cloth, a nonwoven fabric or the like, or by pushing and tearing the artificial nail using a spatula-shaped or stick-shaped tool, without imposing a burden on the fingertip or the nail.

Meanwhile, in regard to the method described above, wiping and push-tearing can be performed while the artificial nail is immersed, and then the artificial nail can be removed more rapidly. Also, wiping/detachment may be accelerated by applying ultrasonic waves, vibration or the like.

Furthermore, the artificial nail removal method of the invention may also include other known processes.

### (Nail art kit)

The nail art kit of the invention includes the artificial nail composition of the invention and a removal liquid, and the removal liquid is an aqueous solution at pH 8 to 11.

Preferred embodiments of the artificial nail composition of the invention and the removal liquid in the nail art kit of the invention are as described above.

Furthermore, the nail art kit of the invention may include optional articles in addition to the artificial nail composition and the removal liquid described above.

Examples thereof include an artificial nail composition other than the artificial nail composition of the invention, such as an artificial nail composition for a color layer or a top layer; a nail file such as a file; an ink brush such as a flat brush, or a paint brush, for applying the artificial nail composition; an exposure apparatus such as a UV light; a wiping or cleaning liquid; a wipe for wiping; a nail brush; a dust brush; a nail form used for repair of the nail, decorative stones made of acryl, glass, metal, or natural stone; nail seal; decorative powder such as glitter or hologram; a cutter; a spatula; a stick; and a separator for separating the distances between fingers in order to prevent the contact between nails. However, the articles are not limited to these.

### EXAMPLES

Hereinafter, the invention will be more specifically explained by way of Examples, but the invention is not intended to be limited to the embodiments in these Examples. In addition, unless particularly stated otherwise, "parts" and "percent (%)" are on a mass basis.

### (Examples 1 to 7 and Comparative Example 1)

Various components indicated in the following Table 1 are uniformly mixed, and artificial nail compositions (Samples 1 to 8) were obtained. Meanwhile, the numbers in the table mean the parts by mass added, and the symbol "-" means that the relevant component is not included.

**[Table 1]**

| Incorporated material | Number of atoms between C=C group and COOH group | Sample 1 | Sample 2 | Sample 3 | Sample 4 | Sample 5 | Sample 6 | Sample 7 | Sample 8 |
|---|---|---|---|---|---|---|---|---|---|
| 2-Acryloyloxyethylsuccinic acid (a-1) | 8 | 39 | - | - | - | - | - | - | - |
| 6-Acryloyloxyhexanoate (a-2) | 7 | - | 39 | - | - | - | - | - | - |
| (2-Acryloyloxy)ethoxyacetic acid (a-3) | 6 | - | - | 39 | - | - | - | | - |
| 4-Acryloyloxybutanoic acid (a-4) | 5 | - | - | - | 39 | - | - | - | - |
| β-carboxyethyl acrylate (a-5) | 4 | - | - | - | - | 39 | - | - | - |
| 2-Acryloyloxyacetic acid (a-6) | 3 | - | - | - | - | - | 39 | | - |
| Acrylic acid (a-7) | 0 | - | - | - | - | - | - | 39 | - |
| 2-Hydroxyethyl methacrylate (a-8) | No COOH | - | - | - | - | - | - | - | 39 |
| I-Hydroxycyclohexyl phenyl ketone | | 1 | 1 | 1 | 1 | 1 | 1 | 1 | 1 |
| Polyurethane methacrylate 1 below | | 50 | 50 | 50 | 50 | 50 | 50 | 50 | 50 |
| Methyl ethyl ketone | | 10 | 10 | 10 | 10 | 10 | 10 | 10 | 10 |

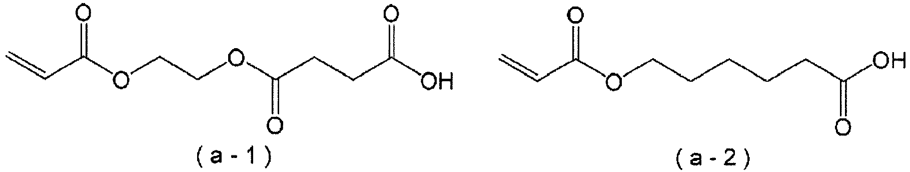

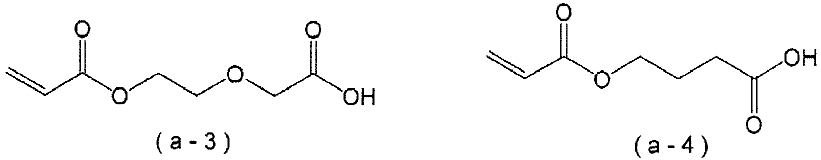

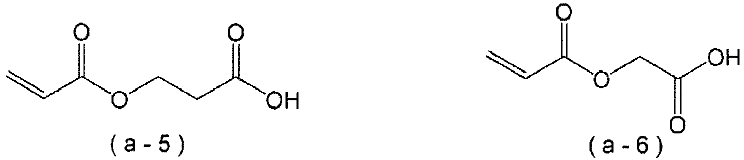

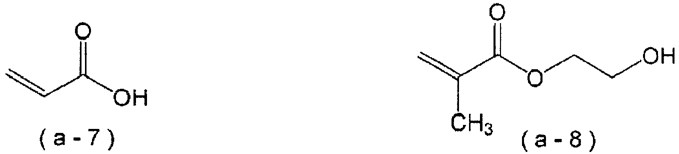

Polyurethane methacrylate 1 in Table 1 was obtained by the following method.

The compound was obtained by reacting 2.6 parts of 2-hydroxyethyl methacrylate with 111.1 parts of isophorone diisocyanate and 44.2 parts of 1,4-dihydroxybutane. Polyurethane methacrylate 1 (weight average molecular weight measured by GPC and calculated relative to polystyrene standards: 20,000, acid value: 0 mmol/g, C=C value: 0.12 mmol/g).

0.25 g of each of the compositions thus obtained (Samples 1 to 8) was applied on a plastic substrate molded into a nail shape using an ink brush, and the composition was irradiated with a UV lamp (36 W) manufactured by Beauty Nailer Co., Ltd. for 2 minutes. Thereafter, the surface was cleaned with ethanol, and then the composition (coating) was observed by visual inspection. The composition was completely solidified (cured), and exhibited gloss (film thickness: 110 µm).

The surface of each composition (photocured film) of these photocured Samples 1 to 8 was scratched with a file (nail file), and the composition was immersed (brought into contact) in a removal liquid 1 (liquid temperature: 40°C) described below, while the duration was varied. Subsequently, each sample (cured film) thus taken out was removed from the substrate by performing pushing and tearing using a stick-shaped tool. At this time, removability was evaluated based on the immersion (contact) time required to remove the composition. The results are presented in Table 2.

Removal liquid 1 composition (pH = 9.9)
· Water: 9379.8 parts
· Sodium carbonate: 130.0 parts
· Sodium hydrogen carbonate: 70.0 parts
· NEWCOL B 13: 500.0 parts
· Monobasic ammonium phosphate: 20.0 parts
· 2-Bromo-2-nitropropane-1,3-diol: 0.1 parts
· 2-Methyl-4-isothiazolin-3-one: 0.1 parts

NEWCOL B13 used was polyoxyalkylene aryl ether (manufactured by Nippon Nyukazai Co., Ltd.).

**[Table 2]**

| Example/Comparative Example | Example 1 | Example 2 | Example 3 | Example 4 | Example 5 | Example 6 | Example 7 | Comparati ve Example 1 |
|---|---|---|---|---|---|---|---|---|
| Artificial nail compositio n | Sample 1 | Sample 2 | Sample 3 | Sample 4 | Sample 5 | Sample 6 | Sample 7 | Sample 8 |
| Removability (min) | 7.5 | 8 | 8.5 | 8.5 | 9 | 10 | 13 | >15 |

As is obvious from the results of Table 2, Sample 8 that did not contain a monomer having an acid group could not be removed with an aqueous solution at pH 8 to 11 within 15 minutes, while Samples 1 to 7 that contained a monomer having an acid group could be removed in a short period of time using an aqueous solution at pH 8 to 11 that was within the scope of the invention.

### (Examples 8 to 14 and Comparative Example 2)

Various components indicated in the following Table 3 were uniformly mixed, and artificial nail compositions (Samples 9 to 16) were obtained. Meanwhile, the numbers in the table mean the parts by mass added, and the symbol "-" means that the relevant component is not included. Also, the copolymerization ratios of the polymers used are molar ratios.

**[Table 3]**

| Incorporated material | Sample 9 | Sample 10 | Sample 11 | Sample 12 | Sample 13 | Sample 14 | Sample 15 | Sample 16 |
|---|---|---|---|---|---|---|---|---|
| Polymer 1 (Mw: 21,000) | 50 | - | - | - | - | - | - | - |
| Polymer 2 (Mw: 20,000) | - | 50 | - | - | - | - | - | - |
| Polymer 3 (Mw: 16,000) | - | - | 50 | - | - | - | - | - |
| Polymer 4 (Mw: 17,000) | - | - | - | 50 | - | - | - | - |
| Polymer 5 (Mw: 20,000) | - | - | - | - | 50 | - | - | - |
| Polymer 6 (Mw: 17,000) | - | - | - | - | - | 50 | - | - |
| Polymer 7 (Mw: 22,000) | - | - | - | - | - | - | 50 | - |
| Polymer 8 (Mw: 19,000) | - | - | - | - | - | - | - | 50 |
| 2-Hvdroxyethyl methacrylate | 39 | 39 | 39 | 39 | 39 | 39 | 39 | 39 |
| I-Hydroxycyclohexyl phenyl ketone | 1 | 1 | 1 | 1 | 1 | 1 | 1 | 1 |
| Methyl ethyl ketone | 10 | 10 | 10 | 10 | 10 | 10 | 10 | 10 |

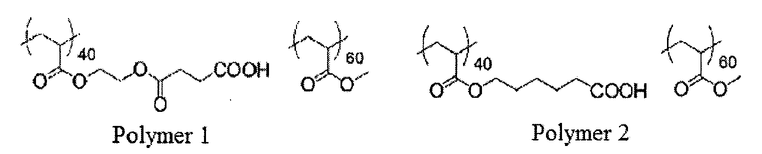

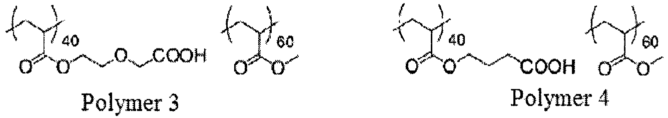

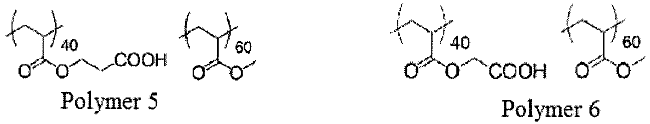

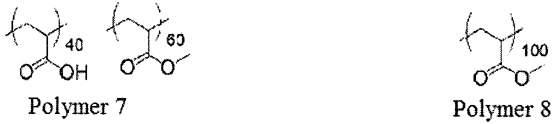

0.25 g of each of the compositions thus obtained (Samples 9 to 16) was applied on a plastic substrate molded into a nail shape using an ink brush, and the composition was irradiated with a UV lamp (36 W) manufactured by Beauty Nailer Co., Ltd. for 2 minutes. Thereafter, the surface was cleaned with ethanol, and then the composition (coating) was observed by visual inspection. The composition was completely solidified (cured), and exhibited glossiness (film thickness: 115 µm).

The surface of each composition (photocured film) of these photocured Samples 9 to 16 was scratched with a file (nail file), and the composition was immersed (brought into contact) in a removal liquid 2 (liquid temperature: 40°C) described below, while the duration was varied. Subsequently, each sample (cured film) thus taken out was removed from the substrate by performing pushing and tearing using a stick-shaped tool. At this time, removability was evaluated based on the immersion (contact) time required to remove the composition. The results are presented in Table 4.

Removal liquid 2 composition (pH = 9.4)
· Water: 8349.8 parts
· Sodium carbonate: 60.0 parts
· Sodium hydrogen carbonate: 240.0 parts
· PIONIN B 111: 400.0 parts
· Methyl cellulose: 950.0 parts
· 2-Bromo-2-nitropropane-1,3-diol: 0.1 parts
· 2-Methyl-4-isothiazolin-3-one: 0.1 parts

The following products were used as PIONIN B111 and methyl cellulose described above.
· PIONIN B111: Lauryltrimethylammonium chloride (manufactured by Takemoto Oil & Fat Co., Ltd.)
· Methyl cellulose: methyl cellulose (manufactured by Shin-Etsu Chemical Co., Ltd.)

**[Table 4]**

| Example/Comparative Example | Example 8 | Example 9 | Example 10 | Example 11 | Example 12 | Example 13 | Example 14 | Comparative Example 2 |
|---|---|---|---|---|---|---|---|---|
| Artificial nail composition | Sample 9 | Sample 10 | Sample 11 | Sample 12 | Sample 13 | Sample 14 | Sample 15 | Sample 16 |
| Removability (min) | 8.5 | 9 | 9.5 | 10 | 10.5 | 11 | 14 | >20 |

As is obvious from the results of Table 4, Sample 16 that did not contain a polymer having an acid group could not be removed with an aqueous solution at pH 8 to 11 within 20 minutes, while Samples 9 to 15 that contained a polymer having an acid group could be removed in a short period of time using an aqueous solution at pH 8 to 11 that was within the scope of the invention.

### (Examples 15 to 24 and Comparative Example 3)

Various components indicated in the following Table 5 were uniformly mixed, and artificial nail compositions (Samples 17 to 27) were obtained. Meanwhile, the numbers in the table mean the parts by mass added, and the symbol "-" means that the relevant component is not included.

**[Table 5]**

| Incorporated material | Sample 17 | Sample 18 | Sample 19 | Sample 20 | Sample 21 | Sample 22 | Sample 23 | Sample 24 | Sample 25 | Sample 26 | Sample 27 |
|---|---|---|---|---|---|---|---|---|---|---|---|
| Polyurethane-1 | 50 | - | - | - | - | - | - | - | - | - | - |
| Polyurethane-2 | - | 50 | - | - | - | - | - | - | - | - | - |
| Polyurethane-3 | - | - | 50 | - | - | - | - | - | - | - | - |
| Polyurethane-4 | - | - | - | 50 | - | - | - | - | - | - | - |
| Polyurethane-5 | - | - | - | - | 50 | - | - | - | - | - | - |
| Polvurethane-6 | - | - | - | - | - | 50 | - | - | - | - | - |
| Polyurethane-7 | - | - | - | - | - | - | 50 | - | - | - | - |
| Polyurethane-8 | - | - | - | - | - | - | - | 50 | - | - | - |
| Polyurethane-9 | - | - | - | - | - | - | - | - | 50 | - | - |
| Polyurethane-10 | - | - | - | - | - | - | - | - | - | 50 | - |
| Polyurethane-11 | - | - | - | - | - | - | - | - | - | - | 50 |
| 1-Hydroxycyclohexyl phenyl ketone | 1 | 1 | 1 | 1 | 1 | 1 | 1 | 1 | 1 | 1 | 1 |
| 2-Hydroxyethyl methacrylate | 39 | 39 | 39 | 39 | 39 | 39 | 39 | 39 | 39 | 39 | 39 |
| Methyl ethyl ketone | 10 | 10 | 10 | 10 | 10 | 10 | 10 | 10 | 10 | 10 | 10 |

Polyurethane-1 to polyurethane-11 used therein are as follows.

### <Polyurethane-1>

Polyurethane obtainable by reacting 1.20 parts of isopropyl alcohol with 144.55 parts of tolylene diisocyanate and 109.99 parts of 2,2-dimethylolpropionic acid (weight average molecular weight measured by GPC and calculated relative to polystyrene standards: 27,000, acid value: 3.2 mmol/g)

### <Polyurethane-2>

Polyurethane obtainable by reacting 1.20 parts of isopropyl alcohol with 134.10 parts of tolylene diisocyanate and 124.01 parts of compound A having a structure described below (weight average molecular weight measured by GPC and calculated relative to polystyrene standards: 26,000, acid value: 2.9 mmol/g)

### <Polyurethane-3>

Polyurethane obtainable by reacting 1.20 parts of isopropyl alcohol with 134.10 parts of tolylene diisocyanate and 134.67 parts of compound B having a structure described below (weight average molecular weight measured by GPC and calculated relative to polystyrene standards: 26,000, acid value: 2.8 mmol/g)

### <Polyurethane-4>

Polyurethane obtainable by reacting 1.20 parts of isopropyl alcohol with 123.65 parts of tolylene diisocyanate and 143.65 parts of compound C having a structure described below (weight average molecular weight measured by GPC and calculated relative to polystyrene standards: 27,000, acid value: 2.6 mmol/g)

### <Polyurethane-5>

Polyurethane obtainable by reacting 1.20 parts of isopropyl alcohol with 134.10 parts of tolylene diisocyanate and 153.46 parts of compound D having a structure described below (weight average molecular weight measured by GPC and calculated relative to polystyrene standards: 28,000, acid value: 2.5 mmol/g)

### <Polyurethane-6>

Polyurethane obtainable by reacting 1.20 parts of isopropyl alcohol with 116.69 parts of tolylene diisocyanate and 153.95 parts of compound E having a structure described below (weight average molecular weight measured by GPC and calculated relative to polystyrene standards: 27,000, acid value: 2.4 mmol/g)

### <Polyurethane-7>

Polyurethane obtainable by reacting 1.20 parts of isopropyl alcohol with 134.10 parts of tolylene diisocyanate and 163.21 parts of compound F having a structure described below (weight average molecular weight measured by GPC and calculated relative to polystyrene standards: 28,000, acid value: 2.3 mmol/g)

### <Polyurethane-8>

Polyurethane obtainable by reacting 1.20 parts of isopropyl alcohol with 174.16 parts of tolylene diisocyanate and 89.22 parts of 1,4-butanediol (weight average molecular weight measured by GPC and calculated relative to polystyrene standards: 26,000, acid value: 0 mmol/g)

### <Polyurethane-9>

Polyurethane obtainable by reacting 1.20 parts of isopropyl alcohol with 134.10 parts of tolylene diisocyanate and 126.18 parts of compound G having a structure described below (weight average molecular weight measured by GPC and calculated relative to polystyrene standards: 25,000, acid value: 2.7 mmol/g)

### <Polyurethane-10>

Polyurethane obtainable by reacting 1.20 parts of isopropyl alcohol with 134.10 parts of tolylene diisocyanate and 126.83 parts of compound H having a structure described below (weight average molecular weight measured by GPC and calculated relative to polystyrene standards: 26,000, acid value: 2.7 mmol/g)

### <Polyurethane-11>

Polyurethane obtainable by reacting 1.20 parts of isopropyl alcohol with 134.10 parts of tolylene diisocyanate and 163.86 parts of compound I having a structure described below (weight average molecular weight measured by GPC and calculated relative to polystyrene standards: 28,000, acid value: 2.3 mmol/g)

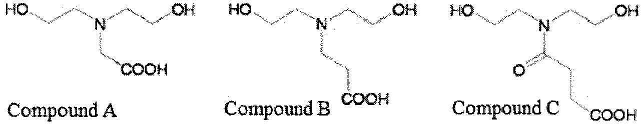

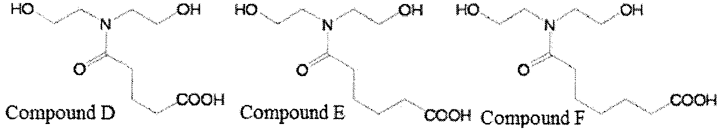

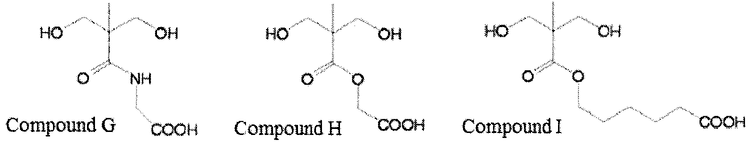

0.25 g of each of the compositions thus obtained was applied on a plastic substrate molded into a nail shape using an ink brush, and the composition was irradiated with a UV lamp (36 W) manufactured by Beauty Nailer Co., Ltd. for 2 minutes. Thereafter, the surface was cleaned with ethanol, and then the composition (coating) was observed by visual inspection. The composition was completely solidified (cured), and exhibited gloss (film thickness: 110 µm).

The surface of each composition (photocured film) of these photocured Samples 17 to 27 was scratched with a file (nail file), and the composition was immersed (brought into contact) in a removal liquid 3 (liquid temperature: 40°C) described below, while the duration was varied. Subsequently, each sample (cured film) thus taken out was removed from the substrate by performing pushing and tearing using a stick-shaped tool. At this time, removability was evaluated based on the immersion (contact) time required to remove the composition. The results are presented in Table 6.

Removal liquid 3 composition (pH = 9.7)
· Water: 8079.8 parts
· Sodium carbonate: 120.0 parts
· Sodium hydrogen carbonate: 80.0 parts
· W-2: 750.0 parts
· Monobasic ammonium phosphate: 20.0 parts
· 2-Bromo-2-nitropropane-1,3-diol: 0.1 parts
· 2-Methyl-4-isothiazolin-3-one: 0.1 parts
· Gum arabic: 250.0 parts
· Hydroxyalkylated starch: 700.0 parts
The following products were used as W-2 and hydroxyalkylated starch.
· W-2: Amphoteric surfactant represented by the following formula
· Hydroxyalkylated starch: PENON JE66 (manufactured by Nippon Starch Chemical Co., Ltd.)

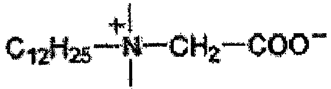

**[Table 6]**

| Example/Comparative Example | Example 15 | Example 16 | Example 17 | Example 18 | Example 19 | Example 20 | Example 21 | Example 22 | Example 23 | Example 24 | Comparative Example 3 |
|---|---|---|---|---|---|---|---|---|---|---|---|
| Artificial nail composition | Sample 17 | Sample 18 | Sample 19 | Sample 20 | Sample 21 | Sample 22 | Sample 23 | Sample 25 | Sample 26 | Sample 27 | Sample 24 |
| Removability (min) | 10.5 | 9.5 | 9 | 8.5 | 8 | 7.5 | 7.5 | 8.5 | 8.5 | 7 | >20 |

As is obvious from the results of Table 6, Sample 24 that did not contain a polyurethane having an acid group could not be removed with an aqueous solution at pH 8 to 11 within 20 minutes, while Samples 17 to 23 that contained a polyurethane having an acid group could be removed in a short period of time using an aqueous solution at pH 8 to 11 that was within the scope of the invention.

### (Example 25 and Comparative Examples 4 and 5)

49 parts of 2-methacryloyloxyethylsuccinic acid, 1 part of 1-hydroxycyclohexyl phenyl ketone, 40 parts of the aforementioned polyurethane-1, and 10 parts of methyl ethyl ketone were uniformly mixed, and an artificial nail composition (Sample 28) was obtained.

A first cured layer was formed on a plastic nail using the prepared sample. Specifically, the entire nail was sanded with a nail file, and then the sample was uniformly applied thereon using a flat brush. Subsequently, the sample was irradiated with UV for 60 seconds using a UV lamp (36 mW) manufactured by Beauty Nailer Co., Ltd. (film thickness 103 µm). BIO S-GEL (trade name) (film thickness 115 µm) manufactured by Bio Sculpture, and then SOAK OFF TYPE CLEAR GEL (trade name) (film thickness 110 µm) manufactured by Ibd Japan-Shinwa Corporation were used thereon and were cured with ultraviolet radiation for 4 minutes and 2 minutes, respectively. Thus, nail decoration 1 in which the artificial nail composition of the invention was used as a base layer on a plastic nail, was formed.

Similarly, a first layer (film thickness 105 µm) was formed on a plastic nail using CLEAR GEL (trade name) manufactured by Bio Sculpture. Subsequently, BIO S-GEL (trade name) (film thickness 113 µm) manufactured by Bio Sculpture, and then SOAK OFF TYPE CLEAR GEL (trade name) (film thickness 110 µm) manufactured by Ibd Japan-Shinwa Corporation were used thereon and were cured with ultraviolet radiation for 4 minutes and 2 minutes, respectively. Thus, nail decoration 2 in which a comparative gel nail agent was used as a base layer on a plastic nail was formed.

The nail decorations 1 and 2 thus obtained were subjected to the following evaluations. The results are presented in Table 7.

### <Evaluation of adhesiveness>

Adhesiveness of the nail decoration was evaluated as follows, after a cycle of immersing the decorated nail in a warm water bath at 40°C for 5 minutes, subsequently taking out the nail, and cooling and drying the nail for 1 minute, was repeated 12 times.
A: Floating, peeling and the like of the film do not occur.
B: Floating of the film is observed, but peeling does not occur.
C: Peeling of the film is observed.

### <Evaluation of removability>

### Removability of the nail decorations was evaluated as follows.

The surface of each of these photocured samples (photocured film) was scratched with a file (nail file), and the sample was immersed (brought into contact) in a removal liquid 4 (liquid temperature: 40°C) having a composition described below, while the duration was varied. Subsequently, each sample (cured film) thus taken out was removed from the substrate by performing pushing and tearing using a stick-shaped tool. At this time, removability was evaluated based on the immersion (contact) time required to remove the sample.

On the other hand, in Comparative Example 5, an evaluation of removability using conventional cotton soaked with acetone was also carried out for the nail decoration 2. Specifically, the nail decoration 2 was wrapped with cotton soaked with acetone, and then the cotton soaked with acetone and the nail decoration were wrapped with aluminum foil. The nail decoration 2 was taken out after different contact times, and the nail decoration was removed from the substrate by performing pushing and tearing on the nail decoration using a stick-shaped tool. At this time, removability was evaluated based on the contact time required to remove the nail decoration.

Removal liquid 4 (pH: 9.6)
· Water: 8,150 parts
· Sodium carbonate: 160 parts
· Sodium hydrogen carbonate: 160 parts
· PIONIN C-157-K: 500 parts
· Polyvinylpyrrolidone: 850 parts
· Soda hexametaphosphate: 180 parts

PIONIN C157K was a product such as follows.
· PIONIN C157K: Betaine type surfactant, manufactured by Takemoto Oil & Fat Co., Ltd.

**[Table 7]**

| | Sample | Removal liquid | Adhesiveness | Removability (min) |
|---|---|---|---|---|
| Example 25 | Nail decoration 1 | Removal liquid 4 | A | 12 |
| Comparative Example 4 | Nail decoration 2 | Removal liquid 4 | A | >40 |
| Comparative Example 5 | Nail decoration 2 | Acetone | A | 25 |

As is obvious from the results of Table 7, the nail decoration 2 in which a comparative artificial nail composition was used for the base layer could not be removed even if the nail decoration was immersed in the removal liquid 4 for 40 minutes. Furthermore, even in the case of removal using conventional cotton soaked with acetone, a contact time of 25 minutes was required. On the contrary, it was found that the nail decoration 1 formed from an artificial nail composition of the invention containing a compound having an ethylenically unsaturated group and an acid group, could be removed by immersion for 12 minutes using the removal liquid 4.

From these results, it was found that the first cured layers formed from the compositions of Examples have satisfactory adhesiveness and excellent removability.

### (Example 26)

49 parts of 2-methacryloyloxyethylsuccinic acid, 1 part of 1-hydroxycyclohexyl phenyl ketone, 40 parts of the polymer 1, and 10 parts of methyl ethyl ketone were uniformly mixed, and an artificial nail composition (Sample 29) was obtained.

A first cured layer was formed on a plastic nail using the prepared sample. Specifically, the entire nail was sanded with a nail file, and then the sample was uniformly applied thereon using a flat brush. Subsequently, the sample was irradiated with UV for 60 seconds using a UV lamp (36 mW) manufactured by Beauty Nailer Co., Ltd. (film thickness 110 µm). BIO S-GEL (trade name) (film thickness 105 µm) manufactured by Bio Sculpture, and then SOAK OFF TYPE CLEAR GEL (trade name) (film thickness 110 µm) manufactured by Ibd Japan-Shinwa Corporation were used thereon and were cured with ultraviolet radiation for 4 minutes and 2 minutes, respectively. Thus, a nail decoration in which the artificial nail composition of the invention was used as a base layer on a plastic nail, was formed.

The nail decoration thus obtained was subjected to the following evaluation.

A cycle of immersing the nail decoration in a warm water bath at 40°C for 5 minutes, subsequently taking out the nail decoration, and cooling and drying the nail decoration for 1 minute, was repeated 12 times. However, floating and peeling of the film did not occur.

On the other hand, the surface of the nail decoration thus obtained was scratched with a file (nail file), the surface was wrapped with cotton soaked with the removal liquid 4 (liquid temperature: 40°C), and the nail decoration and the cotton were brought into contact while the duration was varied. Subsequently, the sample (cured film) thus taken out was removed from the substrate by performing pushing and tearing using a stick-shaped tool. At this time, the contact time required for removal was 10 minutes.

The surface of the nail decoration thus obtained was scratched with a file (nail file), and the surface was brought into contact with the removal liquid 4 by spraying the removal liquid 4 using a sprayer at an interval of once per minute. Subsequently, the sample (cured film) thus taken out was removed from the substrate by performing pushing and tearing using a stick-shaped tool. At this time, the number of times of spraying required for removal was 10 times.

The surface of the nail decoration thus obtained was scratched with a file (nail file), and the nail decoration was immersed in the removal liquid 4 while the duration of immersion was varied. Subsequently, the sample (cured film) thus taken out was removed from the substrate by performing pushing and tearing using a stick-shaped tool. At this time, the immersion time required for removal was 10 minutes.

As is obvious from the above results, it was found that a nail decoration obtained using the artificial nail composition of the invention can be removed in 10 minutes using the relevant removal liquid.

From these results, it was found that the first cured layers formed from the compositions of Examples have satisfactory adhesiveness and excellent removability.

### (Example 27)

49 parts of 2-methacryloyloxyethylsuccinic acid, 1 part of 1-hydroxycyclohexyl phenyl ketone, 40 parts of polyurethane-7 described above, and 10 parts of methyl ethyl ketone were uniformly mixed, and a gel nail agent (Sample 30) was obtained.

A first cured layer was formed on a plastic nail using the prepared sample. Specifically, the entire nail was sanded with a nail file, and then the sample was uniformly applied thereon using a flat brush. Subsequently, the sample was irradiated with UV for 60 seconds using a UV lamp (36 mW) manufactured by Beauty Nailer Co., Ltd. (film thickness 113 µm). BIO S-GEL (trade name) (film thickness 115 µm) manufactured by Bio Sculpture, and then SOAK OFF TYPE CLEAR GEL (trade name) (film thickness 110 µm) manufactured by Ibd Japan-Shinwa Corporation were used thereon and were applied so as to cover the base layer as illustrated in Fig. 1. The agents were cured with ultraviolet radiation for 4 minutes and 2 minutes, respectively. Thus, a nail decoration in which the gel nail agent of the invention was used as a base layer on a plastic nail was formed.

The nail decoration thus obtained was subjected to the following evaluation.

A cycle of immersing the nail decoration in a warm water bath at 40°C for 5 minutes, subsequently taking out the nail decoration, and cooling and drying the nail decoration for 1 minute, was repeated 12 times. However, floating and peeling of the film did not occur.

The surface of the nail decoration thus obtained was scratched with a file (nail file), and the nail decoration was immersed in the removal liquid 4 (liquid temperature 40°C), while the duration of immersion was varied. Subsequently, the sample (cured film) thus taken out was removed from the substrate by performing pushing and tearing using a stick-shaped tool. At this time, the immersion time required for removal was 5 minutes.

As is obvious from the above results, it was found that the nail decoration obtained using the gel nail agent of the invention can be removed in 5 minutes using the relevant removal liquid.

From these results, it was found that the first cured layers formed from the composition of Examples have satisfactory adhesiveness and excellent removability.

### (Examples 28 to 34)

Various components indicated in the following Table 8 were uniformly mixed, and artificial nail compositions (Samples 31 to 33) were obtained. Meanwhile, the numbers in the table mean the parts by mass added, and the symbol "-" means that the relevant component is not included.

**[Table 8]**

| Incorporated material | Sample 31 | Sample 32 | Sample 33 |
|---|---|---|---|
| 2-Methacryloyloxyethylsuccinic acid | 49 | - | 49 |
| 2-Hydroxyethyl methacrylate | - | 49 | - |
| Polymer 1 | - | 40 | 40 |
| Polymer 8 | 40 | - | - |
| 1-Hydroxycyclohexyl phenyl ketone | 1 | 1 | 1 |
| Methyl ethyl ketone | 10 | 10 | 10 |

0.25 g of each of the compositions (samples) thus obtained was applied on a plastic substrate molded into a nail shape using an ink brush, and the composition was irradiated with a UV lamp (36 W) manufactured by Beauty Nailer Co., Ltd. for 2 minutes. Thereafter, the surface was cleaned with ethanol, and then the composition (coating) was observed by visual inspection. The composition was completely solidified (cured), and exhibited glossiness (film thickness: 130 µm).

The surface of each composition (photocured film) of these photocured Samples 31 to 33 was scratched with a file (nail file), and the composition was immersed (brought into contact) in removal liquids 5 to 11 (liquid temperature: 40°C) described below, while the duration was varied. Subsequently, each sample (cured film) thus taken out was removed from the substrate by performing pushing and tearing using a stick-shaped tool. At this time, removability was evaluated based on the immersion (contact) time required for removal. The results are presented in Table 9.

The removal liquids 5 to 11 used therein are as follows.

### <Removal liquid 5>

Aqueous solution (pH = 9.2) obtained by adding 6.18 g of boric acid and 2.00 g of sodium hydroxide to 900 mL of ion-exchanged water, dissolving the components, and then adjusting the total amount to 1,000 mL.

### <Removal liquid 6>

Aqueous solution (pH = 9.6) obtained by adding 1.24 g of monoethanolamine and 0.6 g of glacial acetic acid to 900 mL of ion-exchanged water, dissolving the components, and then adjusting the total amount to 1,000 mL.

### <Removal liquid 7>

Aqueous solution (pH = 9.2) obtained by adding 0.68 g of sodium carbonate and 3.68 g of sodium hydrogen carbonate to 900 mL of ion-exchanged water, dissolving the components, and then adjusting the total amount to 1,000 mL.

### <Removal liquid 8>

Aqueous solution (pH = 8.0) obtained by adding 2.1 g of sodium hydrogen carbonate to 900 mL of ion-exchanged water, dissolving the component, and then adjusting the total amount to 1,000 mL.

### <Removal liquid 9>

Aqueous solution (pH = 8.1) obtained by adding 0.52 g of sodium dihydrogen phosphate and 22.68 g of disodium hydrogen phosphate dodecahydrate to 900 mL of ion-exchanged water, dissolving the components, and then adjusting the total amount to 1,000 mL.

### <Removal liquid 10>

Aqueous solution obtained by adding 12.1 g of trishydroxymethylaminomethane to 900 mL of ion-exchanged water, dissolving the component, subsequently adjusting the solution to pH = 10 using 1 M hydrochloric acid, and adjusting the total amount to 1,000 mL.

### <Removal liquid 11>

Aqueous solution obtained by adding 7.73 g of boric acid to 900 mL of ion-exchanged water, dissolving the component, subsequently adjusting the solution to pH = 8.3 using a 2 M aqueous solution of sodium hydroxide, and then adjusting the total amount to 1,000 mL.

**[Table 9]**

| | Removal liquid | | Removability (min) | | |
|---|---|---|---|---|---|
| | No. | pH | Sample 31 | Sample 32 | Sample 33 |
| Example 28 | 5 | 9.2 | 15 | 20 | 10 |
| Example 29 | 6 | 9.6 | 15 | 20 | 10 |
| Example 30 | 7 | 9.2 | 10 | 15 | 5 |
| Example 31 | 8 | 8.0 | 20 | 25 | 15 |
| Example 32 | 9 | 8.1 | 25 | 25 | 20 |
| Example 33 | 10 | 10.0 | 15 | 20 | 15 |
| Example 34 | 11 | 8.3 | 25 | 25 | 20 |

### (Examples 35 and 36, and Comparative Examples 6 and 7)

Various components indicated in the following Table 10 were uniformly mixed, and artificial nail compositions (Samples 34 to 37) were obtained. Meanwhile, the numbers in the table mean the parts by mass added, and the symbol "-" means that the relevant component is not included.

**[Table 10]**

| Incorporated material | Sample 34 | Sample 35 | Sample 36 | Sample 37 |
|---|---|---|---|---|
| Polymer 1 | 70 | - | - | - |
| Polymer 8 | - | - | 70 | - |
| pnlyurethane-7 | - | 70 | - | - |
| Polyurethane-8 | - | - | - | 70 |
| Methyl ethyl ketone | 30 | 30 | 30 | 30 |

0.25 g of each of the compositions thus obtained was applied on a plastic substrate molded into a nail shape using an ink brush, and the composition was dried by applying hot air using a dryer. The composition (coating) was observed by visual inspection, and the composition was completely solidified and exhibited gloss (film thickness: 200 µm).

The surface of each composition (dried film) of these dried Samples 34 to 37 was scratched with a file (nail file), and the composition was immersed (brought into contact) in the removal liquid 3 (liquid temperature: 40°C) described above, while the duration was varied. At this time, removability was evaluated based on the immersion (contact) time required for removal. The results are presented in Table 11.

**[Table 11]**

| | Example 35 | Example 36 | Comparative Example 6 | Comparative Example 7 |
|---|---|---|---|---|
| | Sample 34 | Sample 35 | Sample 36 | Sample 37 |
| Removability (min) | 5 | | >40 | >40 |

### Industrial Applicability

The nail decoration of the invention has satisfactory adhesiveness to the nail, and can be easily removed using a safe aqueous solution. Therefore, the nail decoration can be used for the decoration of a wide variety of objects for decoration, including the nail.

### Explanation of References

1: nail
2: base layer
3: color layer
4: top layer

## Claims

1. An artificial nail removal method comprising:
a step of applying an artificial nail composition on or above the nail of a human being or an animal, or on or above a support to form a coating film;
a step of drying and/or exposing to light the coating film, and thereby forming an artificial nail; and
a removal step of removing the artificial nail by bringing the artificial nail into contact with a removal liquid,
wherein the artificial nail composition includes (Component A) a compound having an ethylenically unsaturated group and an acid group, and/or (Component B) a polymer having an acid group, and
the removal liquid is an aqueous solution having a pH of from 8 to 11.

2. The artificial nail removal method according to claim 1, wherein Component A is represented by the following Formula (1): 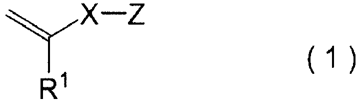 wherein in Formula (1), R¹ represents a hydrogen atom or a monovalent organic group; X represents a single bond or a divalent linking group; and Z represents an acid group.

3. The artificial nail removal method according to claim 2, wherein in the above Formula (1), Z represents a carboxylic acid group.

4. The artificial nail removal method according to any one of claims 1 to 3, wherein Component B is an acrylic polymer and/or a urethane-based polymer.

5. The artificial nail removal method according to any one of claims 1 to 4, wherein Component B is a polymer having a carboxylic acid group.

6. The artificial nail removal method according to claim 5, wherein the polymer having a carboxylic acid group is an acrylic polymer containing a monomer unit represented by the following Formula (2): 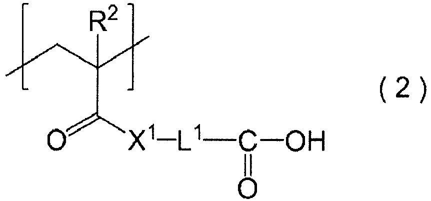 wherein in Formula (2), R² represents a hydrogen atom, a methyl group, or -CH₂Y¹; Y¹ represents a hydroxyl group, a halogeno group, -OC(=O)R³, or -NR³C(=O)R⁴; X¹ represents -O- or -NR³-; R³ and R⁴ each represent a hydrogen atom, an alkyl group having 1 to 6 carbon atoms, or an aryl group having 6 to 12 carbon atoms; and L¹ represents a divalent linking group.

7. The artificial nail removal method according to claim 5, wherein the polymer having a carboxylic acid group is a urethane-based polymer having a monomer unit represented by the following Formula (3): 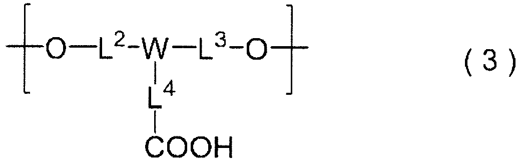 wherein in Formula (3), L², L³ and L⁴ each independently represent a divalent linking group; W represents N or CR³; and R³ represents a hydrogen atom or a monovalent group.

8. The artificial nail removal method according to any one of claims 1 to 7, wherein the removal liquid includes a pH buffering agent.

9. The artificial nail removal method according to claim 8, wherein the pH buffering agent includes at least one selected from the group consisting of a carbonate, a hydrogen carbonate, a phosphate, a borate, and an organic amine compound.

10. The artificial nail removal method according to claim 8 or 9, wherein the pH buffering agent includes a carbonate and a hydrogen carbonate.

11. The artificial nail removal method according to any one of claims 1 to 10, wherein the removal step is a step of removing the artificial nail by immersing the artificial nail in a removal liquid.

12. The artificial nail removal method according to any one of claims 1 to 10, wherein the removal step is a step of removing the artificial nail by spraying a removal liquid to the artificial nail.

13. The artificial nail removal method according to any one of claims 1 to 10, wherein the removal step is a step of removing the artificial nail by wrapping the artificial nail using cotton soaked with a removal liquid.

14. The artificial nail removal method according to any one of claims 1 to 13, wherein the artificial nail composition is used as a base layer.

15. An artificial nail composition comprising (Component A') a compound represented by the following Formula (1'): 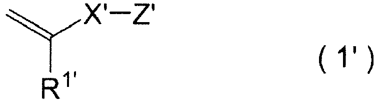 wherein in Formula (1'), R^{1'} represents a hydrogen atom or a monovalent organic group; X' represents a divalent linking group; the minimum number of atoms connecting Z' with the carbon atom to which R^{1'} is bonded is 5 or more; and Z' represents an acid group.

16. The artificial nail composition according to claim 15, wherein in the above Formula (1'), Z' represents a carboxylic acid group.

17. An artificial nail composition comprising (Component B) a polymer having an acid group.

18. The artificial nail composition according to claim 17, wherein Component B is an acrylic polymer and/or a urethane-based polymer.

19. The artificial nail composition according to claim 17 or 18, wherein Component B is an acrylic polymer containing a monomer unit represented by the following Formula (2): 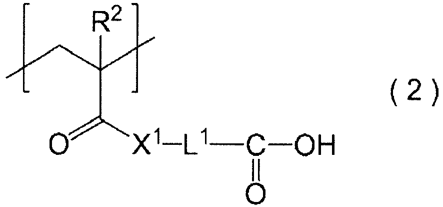 wherein in Formula (2), R² represents a hydrogen atom, a methyl group, or -CH₂Y¹; Y¹ represents a hydroxyl group, a halogeno group, -OC(=O)R³, or -NR³C(=O)R⁴; X¹ represents -O- or -NR³-; R³ and R⁴ each represent a hydrogen atom, an alkyl group having 1 to 6 carbon atoms, or an aryl group having 6 to 12 carbon atoms; and L¹ represents a divalent linking group.

20. The artificial nail composition according to claim 17 or 18, wherein Component B is a urethane-based polymer having a monomer unit represented by the following Formula (3): 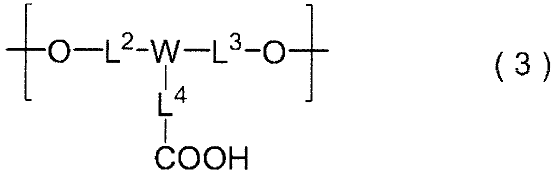 wherein in Formula (3), L², L³, and L⁴ each independently represent a divalent linking group; W represents N or CR³; and R³ represents a hydrogen atom or a monovalent group.

21. The artificial nail composition according to any one of claims 15 to 20, further comprising (Component D) a photopolymerization initiator.

22. An artificial nail comprising a layer formed by drying and/or exposing to light the artificial nail composition according to any one of claims 15 to 21.

23. An artificial nail forming method comprising:
a step of applying the artificial nail composition according to any one of claims 15 to 21 on or above the nail of a human being or an animal, or on or above a support to form a coating film; and
a step of drying and/or exposing to light the coating film, and thereby forming an artificial nail.

24. A nail art kit comprising:
an artificial nail composition; and
a removal liquid,
wherein the artificial nail composition includes (Component A) a compound having an ethylenically unsaturated group and an acid group, and/or (Component B) a polymer having an acid group, and
the removal liquid is an aqueous solution having a pH of from 8 to 11.
